(19) Europäisches Patentamt
European Patent Office
Office européen des brevets

(11) Veröffentlichungsnummer: **0 225 886 B1**

(12) **EUROPÄISCHE PATENTSCHRIFT**

(45) Veröffentlichungstag der Patentschrift: **20.06.90**

(51) Int. Cl.$^5$: **C 07 D 237/20,** A 01 N 43/58

(21) Anmeldenummer: **86902407.5**

(22) Anmeldetag: **18.04.86**

(86) Internationale Anmeldenummer:
**PCT/EP86/00232**

(87) Internationale Veröffentlichungsnummer:
**WO 86/06719 20.11.86 Gazette 86/25**

# (54) NEUE PYRIDAZINIUMVERBINDUNGEN, VERFAHREN ZU DEREN HERSTELLUNG UND DIESE ENTHALTENDE FUNGIZIDE UND ALGIZIDE MITTEL.

(30) Priorität: **15.05.85 DE 3517617**

(43) Veröffentlichungstag der Anmeldung:
**24.06.87 Patentblatt 87/26**

(45) Bekanntmachung des Hinweises auf die Patenterteilung:
**20.06.90 Patentblatt 90/25**

(84) Benannte Vertragsstaaten:
**AT BE CH DE FR GB IT LI LU NL SE**

(56) Entgegenhaltungen:
**EP-A-0 114 770**
**FR-A-1 534 690**

**Chemical Abstracts, Band 67, 1967, Columbus, Ohio, USA H.Lund et al.: "Quaternization reactions.II. Pyridazines" siehe Seite 5089, Zusammenfassung 54091g**

(73) Patentinhaber: **AGROLINZ AGRARCHEMIKALIEN GESELLSCHAFT M.B.H.**
**St. Peterstrasse 25**
**A-4021 Linz (AT)**
(84) **BE CH DE FR GB IT LI LU NL SE AT**

(72) Erfinder: **WÖRTHER, Rudolf Helmut**
**Spaunstrasse 82**
**A-4020 Linz (AT)**
Erfinder: **JELLINGER, Rudolf**
**Im Neugereith 12**
**A-4033 Ebelsberg (AT)**
Erfinder: **BODINGBAUER, Hans Robert**
**Eben 79**
**A-4202 Kirchschlag (AT)**
Erfinder: **GRAF, Josef**
**Kürnberg 116**
**A-4442 Kleinraming (AT)**

(74) Vertreter: **Kunz, Ekkehard, Dr.**
**Chemie Holding AG Patentwesen St. Peter-Strasse 25**
**A-4021 Linz (AT)**

**EP 0 225 886 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

**Beschreibung**

Die Erfindung betrifft neue Pyridaziniumverbindungen, Verfahren zu ihrer Herstellung, sowie diese enthaltende fungizide und algizide Mittel, Verfahren zu deren Herstellung und deren Verwendung.

Aus der DE—OS 2 245 248 sind 3-, 4-, 5-, 6-substituierte Pyridaziniumverbindungen mit blutdrucksteigernder Wirkung bekannt. Einige 3-Phenylpyridaziniumverbindungen und deren Strukturaufklärung wurden in der Acta Chemica Scandinavica, Band 21 (1967), 1067—1080 beschrieben. Etwaige bioligische Eigenschaften der untersuchten Verbindungen sind nicht angegeben. Aus der EP 36 711 sind heterocyclische Verbindungen bekannt, unter anderem auch einige Pyridazine, die aber in 3-Stellung zwingend durch einen 2-Halogenphenylrest substituiert sien müssen. Sie dienen zur Bekämpfung von Akariden, Insekten oder Aphiden, sowie deren Eier und Larven.

Unerwarterweise wurden nun substituierte Pyridaziniumverbindungen gefunden, die ausgezeichnete fungizide und algizide Eigenschaften aufweisen. Diese Verbindungen sind mit Ausnahme der Methyliodide von 6-Methylamino-3-phenylpyridazin, von 6-Dimethylamino-3-phenylpyridazin und von 6-Amino-3-phenylpyridazin neu.

Gegenstand der Erfindung sind demnach Pyridaziniumderivate der allgemeinen Formel I,

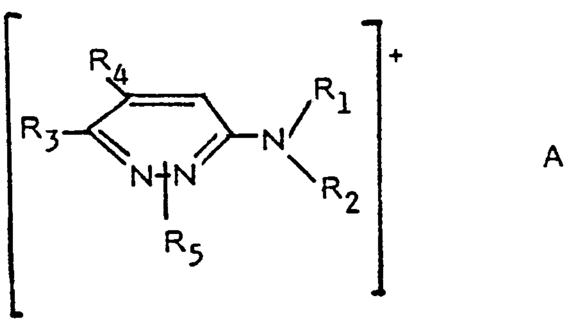

(I)

in der

$R_1$ ein Wasserstoffatom, einen gesättigten oder ungesättigten Alkylrest mit 1—4 C-Atomen,

$R_2$ ein Wasserstoffatom, einen geradkettigen oder Verzweigten, gesättigten oder ungesättigten Alkylrest mit 1—10 C-Atomen, einen Cyclohexylrest, einen Alkoxyalkylrest mit 1—4 C-Atomen in der Alkylkette, einen Hydroxyalkylrest mit 1—4 C-Atomen in der Alkylkette, einen gegebenenfalls ein- oder mehrfach durch Halogenatome substituierten Phenylrest oder

$R_1$ und $R_2$ gemeinsam mit dem N-Atom einen 5—7 gliedrigen Ring bilden, der als weiteres Heteroatom Stickstoff oder Sauerstoff enthalten kann und gegebenenfalls durch Alkyl-, Hydroxyalkyl-, Phenylalkylreste mit 1—4 C-Atomen in der Alkylkette, einen Hydroxyrest oder einen Pyridylrest substituiert ist mit der Maßgabe, daß der Substituent nicht Hydroxy ist, wenn $R_1$ und $R_2$ gemeinsam mit dem N-Atom einen 6 gliedrigen Ring bilden,

$R_3$ einen gegebenenfalls ein- oder mehrfach durch Halogenatome in 3-, 4-oder 5-Stellung, oder gegebenenfalls eine oder mehrfach durch geradket-tige oder verzweigte Alkyl- oder Alkoxygruppen mit 1—4 C-Atomen substituierten Phenylrest oder einen tertiär- Butylrest,

$R_4$ ein Wasserstoffatom oder einen geradkettigen oder verzweigten Alkylrest mit 1—4 C-Atomen,

$R_5$ ein Wasserstoffatom, einen geradkettigen oder verzweigten, gesättigten oder ungesättigten, gegebenenfalls durch Halogen-, Nitril-, Hydroxy-, Alkoxyreste mit 1—4 C-Atomen in der Alkylkette, Phenyl-, Halogenphenyl oder gegebenenfalls halogenierte Phenoxyrest substituierten Alkyl- oder Oxoalkylrest mit 1—20 C-Atomen, oder einen Cyclohexylrest und

$A^-$ ein pflanzenphysiologisch verträgliches Anion bedeutet, mit Ausnahme der Methyliodide von 6-Methylamino-3-phenylpyridazin und 6-Dimethylamino-3-phenylpyridzin und 6-Amino-3-phenylpyridazin.

Weiters wurde gefunden, daß man die erdindungsgemäßen Substanzen erhält, indem man

a) ein Pyridazinverbindung der allgemeinen Formel II

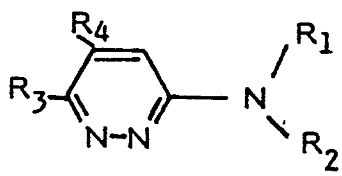

(II)

in der $R_1$, $R_2$, $R_3$, $R_4$ die oben angegebene Bedeutung haben, mit einer Verbindung der allgemeinen Formel

$$R_5Y$$

in der $R_5$ die oben angebenene Bedeutung hat und Y ein Halogenatom oder die Reste Methosulfat oder Tosylat bedeutet, oder

b) ein Verbindung der allgemeinen Formel III

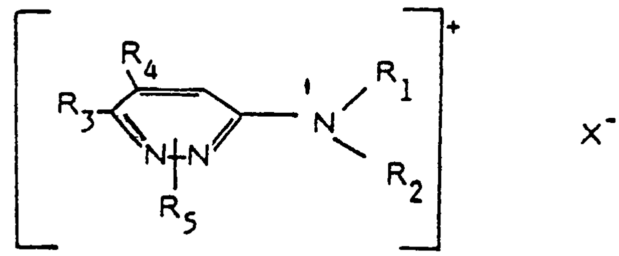

(III)

in der $R_1$, $R_2$, $R_3$, $R_4$ und $R_5$ die oben angegebene Bedeutung haben und X ein Halogenatom bedeutet, mit einem Schwermetallsalz einer anorganischen oder organischen Säure der allgemeinen Formel

Me Z

in der Me ein Schwermetallkation und Z das Anion einer anorganischen oder organischen Säure bedeutet, umsetzt.

Die erfindungsgemäßen Pyridaziniumverbindungen zeigen starke fungizide und algizide Figenschaften und stellen somit eine Bereicherung der Technik dar.

In der Formel I bedeutet $R_1$ ein Wasserstoffatom, einen gesättigten oder ungesättigten Alkylrest mit 1 bis C-Atomen, bevorzugt einen gesättigten Alkylrest mit 1—4 C-Atomen, wie den Methyl-, Ethyl, -Propyl-, oder Butylrest oder den Allylrest.

$R_2$ bedeutet ein Wasserstoffatom, einen gesättigten oder ungesättigten, geradkettigen oder verzweigten Alkylrest mit 1 bis 10 C-Atomen, einen Cycloalkylrest, wie den Cyclohexylrest, einen Alkoxyalkylrest, einen Hydroxyalkylrest, oder einen gegebenenfalls ein- oder mehrfach durch Halogenatome substituierten Phenylrest, wie die verschiedenen Mono-, Di- oder Tri-halogenphenylrest. Bevorzugt sind jene Verbindungen in denen $R_2$ einen geradkettigen oder verzweigten Alkylrest mit 1 bis 4 C-Atomen, einen Alkoxy- oder Hydroxyalkylrest mit 1—4 C-Atomen in den Alkylketten, wie den Methoxyethylrest oder den Hydroxyethylrest, oder den Cyclohexylrest bedeutet.

Weiters können $R_1$ und $R_2$ gemeinsam mit dem Stickstoffatom einen 5 bis 7 gliedrigen heterocyclischen Ring bilden, der als weiteres Heteroatom Stickstoff oder Sauerstoff enthalten kann und gegebenenfalls durch Alkyl- oder Phenylalkylreste oder einen Pyridylrest substituiert ist. Beispiele für solche heterocyclischen Ringe sind gegebenenfalls substituierte Pyrrolidino-, Piperidino-, Morpholino- oder Azacycloheptylringe. Bevorzugt sind jene Verbindungen, in denen $R_1$ und $R_2$ gemeinsam mit dem Stickstoffatom einen 6 oder 7 gliedrigen Ring bilden, wie der Piperidino- oder Azacycloheptylring, der gegebenenfalls ein- oder mehrfach durch Alkylreste mit 1 bis 4 C-Atomen oder durch einen Benzylrest substituiert ist. Besonders bevorzugt sind solche Verbindungen, in denen $R_1$ und $R_2$ gemeinsam mit dem Stickstoffatom einen 6 gliedrigen Ring bilden, der gegebenenfalls ein oder mehrfach durch Alkylrest mit 1 bis 4 C-Atomen oder den Benzylrest substituiert ist.

$R_3$ bedeutet einen gegebenenfalls ein- oder mehrfach durch Halogenatome in 3-, 4- oder 5-Stellung substituierten, oder durch Alkyl oder Alkoxygruppen ein- oder mehrfach substituierten Phenylrest, wie den p-Chlorphenyl- Trimethoxyphenylrest, Butoxyphenyl-, einen Methylphenyl- oder Butylphenylrest oder einen tertiär-Butylrest. Bevorzugt sind solche Verbindungen, in denen $R_3$ einen gegebenenfalls durch Chlor oder geradkettige oder verzweigte Alkylreste substituierte Phenylrest bedeutet. Besonders bevorzugt sind jene Verbindungen in denen $R_3$ einen Phenyl-, einen p-Chlor- oder p-Methylphenylrest bedeutet.

$R_4$ bedeutet ein Wasserstoffatom oder einen geradkettigen oder verzweigten Alkylrest mit 1 bis 4 C-Atomen, vorzugsweise einen Methyl- oder Ethylrest.

Der Rest $R_5$ bedeutet ein Wasserstoffatom, einen geradkettigen oder verzweigten, gesättigten oder ungesättigten gegebenenfalls durch Halogen-, Nitril-, Hydroxy-, Alkoxy-, Phenyl-, Halogenphenyl oder gegebenenfalls halogenierte Phenoxyreste substituierten Alkyl- oder Oxoalkylrest mit 1 bis 20 C-Atomen, zweckmäßig mit 1 bis 15 C-Atomen, bevorzugt mit 1 bis 8 C-Atomen oder einen Cycloalkylrest. Beispiele für solche Reste sind Methyl-, i-Propyl-, i-Butyl-, n-Heptyl-, Octyl-, Nonyl-, Decyl-, Undecyl-, Vinyl-, Allyl-, Butenyl- 2-, 2-Hydroxy-3-phenoxypropyl-, Cyanomethyl, Benzyl-, Monochlorbenzyl-, Dichlorbenzylreste, der Cyclohexylrest u. dgl.

Bevorzugt sind solche Verbindungen, in denen $R_5$ einen gesättigten oder ungesättigten Alkylrest mit 1 bis 8 C-Atomen, besonders bevorzugt einen gesättigten oder ungesättigten Alkylrest mit 1—5 C-Atomen bedeutet.

$A^-$ bedeutet ein pflanzenphysiologisch verträgliches Anion, beispielsweise ein Halogenion wie Chlorid, Bromid, Iodid oder die Anionen Nitrat, Hydrogensulfat, Methosulfat, Trifluormethylsulfonat, Tosylat, Acetat, Trifluoracetat, Lactat, Bezoat, 2-Hydroxybenzoat.

Die Herstellung der Pyridaziniumverbindungen der allgemeinen Formel I kann erfolgen, indem man ein Pyridazin der allgemeinen Formel II mit mindestens einem Mol einer Verbindung der Formel $R_5$ Y umsetzt.

Der Rest $R_5$ geht dabei in Abhängigkeit von der Reaktivität der Ausgangsverbindungen und der Raumerfüllung der Substituenten bevorzugt an das dem Rest $R_4$ benachbarte Stickstoffatom. Es ist aber auch eine Quarternisierung des anderen Stickstoffatoms des Pyridazinringes möglich. Beide Reaktionsprodukte, sowie evt. auftretende Gemische werden erfindungsgemäß beansprucht.

Die Umsetzung kann in Substanz oder in Gegenwart eines inerten Verdünnungsmittels, etwa in einem organischen Verdünnungsmittel wie Aceton, Acetonitril, Dimethylformamid, Nitromethan, N-Methylpyrrolidon erfolgen.

Ferner kann die Reaktion bei Atmosphärendruck oder erhöhtem Druck, etwa bei Drücken von 3 bis 10 bar, durchgeführt werden.

Die Reaktion wird etwa bei Temperaturen von 0 bis 170°C, vorzugsweise bei Temperaturen von 20°C bis zur Rückflüßtemperatur der jeweiligen Reaktionsgemisches durchgeführt.

Die Reaktionsdauer beträgt je nach Temperatur und Reaktivität der Reaktionstellnehmer etwa 30 Minuten bis einige Tage.

Nach beendeter Reaktion werden die flüchtigen Bestandteile des Reaktionsgemisches bei Atmosphärendruck oder vorzugsweise bei vermindertem Druck verdampft, worauf das Endprodukt üblicherweise in großer Reinheit anfällt. Gegebenenfalls kann es aus einem geeigneten Lösungsmittel umkristallisiert werden.

Der Umsatz kann auch in Substanz erfolgen, wobei das Reaktionsgemisch nach beendeter Reaktion etwa in Wasser eingerührt, mit einem organischen Lösungsmittel wie Chloroform, 1,1,1-Trichlorethan, Dichlormethan ein- oder mehrmals extrahiert und der Rücksand nach Verdampfen des Lösungsmittels isoliert und gegebenenfalls umkristallisiert wird.

Die auf diesem Weg erhaltenen Halogensalze können durch Umsalzen in andere Salze mit verschiedenen organischen oder anorganischen Anionen umgewandelt werden.

Hiebei werden die obigen Halogensalze der Formel III mit einem Schwermetallsalz einer anorganischen oder organischen Säure umgesetzt.

Der Umsatz erfolgt beispielsweise in Gegenwart eines inerten Verdünnungsmittels, bevorzugt in Gegenwart von Wasser. Die Reaktion wird etwa bei Temperaturen von 10 bis 80°C, bevorzugt bei Raumtemperatur durchgeführt.

Das praktisch sofort als Niederschlag anfallende Metallhalogenid wird in üblicher Weise, etwa durch Filtrieren oder Zentrifugieren, abgetrennt und die verbleibende Lösung bei Atmosphärendruck oder vorzugsweise bei vermindertem Druck und erhöhter Temperatur von Wasser befreit. Die Substanzen fallen als meist ölige Rückstände in großer Reinheit an.

Die als Ausgangsverbindungen benötigten Pyridazine der Formel II sind teilweise neu und können nach in sich bekannten Methoden erhalten werden, indem man beispielsweise ein 6-Halogenpyridazinverbindung der allgemeinen Formel IV

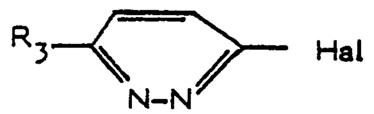

(IV)

in der $R_3$ und $R_4$ wie oben definiert sind und in der Hal ein Halogenatom, vorzugsweise Chlor bedeutet, mit mindestens einem Mol einer Base der allgemeinen Formel V

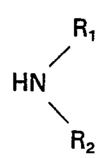

(V)

wobei $R_1$ und $R_2$ wie oben definiert sind, gegebenenfalls in Gegenwart eines inerten Verdünnungsmittels und gegebenenfalls in Gegenwart eines Säureakzeptors umsetzt.

Beispielsweise wurde zur Herstellung von 4-Methyl-3-phenyl-6-piperidinopyridazin, wie folgt verfahren:

a) 30,76 g (0,15 mol) 4-Methyl-3-phenyl-6-chlorpyridazin wurden in 150 ml (1,53 mol) Piperidin gelöst und die Lösung vorsichtig. auf 100°C erhitzt. Nach Abklingen der exothermen Reaktion wurde die Lösung 1,5 Stunden auf Rückflußtemperatur gehalten und anschließend durch Zugabe von Eis auf 20°C gekühlt. Der anfallende Niederschlag wurde isoliert, mit Wasser gewaschen und getrocknet. Nach dem Umkristallisieren aus einem Aceton-Diisopropylethergemisch wurden 36,6 g (0,144 mol) 4-Methyl-3-phenyl-6-piperidinopyridazin mit Fp. 116—118°C erhalten.

b) 10,25 g (0,05 mol) 4-Methyl-3-phenyl-6-chlorpyridazin wurden in 100 ml Xylol augeschlämmt und 6 g Kaliumcarbonat zugegeben. Anschließend wurden 5,1 ml (0,05 mol) Piperidin zugefügt und das Reaktionsgemisch 40 Stunden unter Rückfluß gehalten. Nach Verdampfen des Xylols unter verminderten Druck wurde der Rückstand in Wasser eingerührt. Das anfailende Festprodukt wurde isoliert, mit Wasser

gewaschen und getrocknet. Nach dem Umkristallisieren aus Diisopropylether wurden 11,95 g (0,047 mol) an 4-Methyl-3-phenyl-6-piperidinopyridazin mit Fb. 116—118°C erhalten.

Auf einem der beschriebenen Wege wurden die Ausgangsverbindungen für die beanspruchten Pyridaziniumverbindungen hergestellt.

Die erfindungsgemäßen Mittel können zur Behandlung von Pflanzen oder technischen Materialien eingesetzt werden, die duch Einwirkung von Pilzen, Algen oder Bakterien, wie z.B. Xanthomonasarten, erkrankt sind. Im Pflanzenschutz werden beispielsweise die fungiziden und algiziden Mittel zur Bekämpfung von Schadorganismen aus den Klassen der Schizomyceten, Myxomyceten, Phycomyceten, Ascomyceten, Basidiomyceten und Deuteromyceten und zur Bekämpfung von Algen eingesetzt. Die gute Pflanzenverträglichkeit der Wirkstoffe und die zum Teil systemische Wirksamkeit in den zur Behandlung von Pflanzenkrankheiten notwendigen Konzentrationen erlaubt eine Behandlung von oberirdischen Pflanzenteilen, von Pflanz- und Saatgut und des Bodens. Als Pflanzen seien etwa Getreidearten, wie Weizen, Gerste, Roggen oder Hafer, weiters Reis, Mais, Baumwolle, Soja, Kaffee, Zuckerrohr, sowie Gemüsearten, wie Gurken, Bohnen, Tomaten oder Kürbisgewächse genannt.

Mit besonders gutem Erfolg können die erfindungsgemäßen Mittel etwa zur Bekämpfung folgender Pflanzenkrankheiten eingesetzt werden:

Erysiphe graminis (echter Mehltau) in Getreide
Erysiphe betae an Zuckerrüben,
Erysiphe chichoracearum an Kürbisgewächsen,
Erysiphe polygoni an Bohnen,
Podosphaera leucotricha an Äpfeln,
Sphaerotheca pannosa an Rosen,
Uncinula necator an Reben,
Puccinia-Arten an Getreide,
Ustilago-Arten an Getreide und Zuckerrohr,
Uromyces appendiculatus und Uromyces phaseoli an Bohnen,
Venturia inaequalis an Äpfeln,
Septoria nodorum an Weizen,
Septoria appii an Selleria,
Phytophthora infestans an Kartoffeln und Tomaten,
Plasmopora viticola an Wein,
Pseudocercosporella herpotrichoides an Getreide.

In entsprechenden Aufwandmengen können die Stoffe auch als Holzschutzmittel gegen holzverfärbende Pilze oder Moderfäulepilze eingesetzt werden, wie z.B. zur Bekämpfung von Merulius lacrimans, Polyporus vaporarius Poria placenta oder Polystictus cinnabarinus. Die erfindungsgemäßen Mittel können weiters zur Algen- und Schleimbekämpfung in landwirtschaftlichen Kulturen mit Überflutung, bei Industrieprozessen, in Kühlanlagen und Abwässern, in Gewächshäusern, Gewässern, in Schwimmbecken und Aquarien verwendet werden.

Die Wirkstoffe können je nach ihrem Anwendungsgebiet in die üblichen Formulierungen übergeführt werden, wie Lösungen, Spritzpulver, Emulsionskonzentrate, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.a., sowie ULV-Kalt- und Warmnebel-Formulierungen.

Diese Formulierungen werden bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von Tensiden, also Emulgatoren und/oder Dispergier- und/oder Netzmitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Cyclohexan oder Paraffine, z.B. Erdölfraktionen, Alkohole, wie Butanol oder Glykol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten, gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe, sowie Butan, Propan, Stickstoff und Kohlendioxid; als feste Trägerstoffe kommen in Frage: z.B. natürliche Gesteinsmehle, wie Kaoline, Tonerde, Talkum, Kreide, Quarz, Attapulgit, Montmorillonit oder Diatomeenerde und synthetische Produkte, wie hochdisperse Kieselsäure, Aluminiumoxid und Silikate; als feste Trägerstoffe für Granulate kommen in Frage: z.B. gebrochene und fraktionierte natürliche Gesteine, wie Calcit, Marmor, Bims, Sepiolith, Dolomit sowie synthetische Granulate aus anorganischen und organischen Mehlen, sowie Granulate aus organischem Material, wie Sägemehl, Kokosnuss-Schalen, Maiskolben und Tabakstengel; als Emulgiermittel und/oder schaumerzeugende Mittel kommen in Frage: z.B. nichtionogene und ionogene Tenside, wei Polyoxyethylen-Sorbitan- Tallölester, Na-Oleylmethyltaurid, Polyoxyethylen-Fettsäure-Ester, Polyoxyethylen-Fettalkoholether, z.B. Alkylarylpolyglykolether, Alkylsulfonate, Alkylsulfate, Arylsulfate und Arylalkylsulfonate sowie Eiweisshydrolysate; als

Dispergiermittel kommen in Frage: z.B. Ligninsulfonate oder Kondensationsprodukte von Arylsulfonaten mit Formaldehyd.

Es können in den Formulierungen Haft- und Verdickungsmittel wie Carboxymethylcellulose, Methylcellulose, natürliche und synthetische pulverige, körnige und latexförmige Polymere verwendet werden, wie Gummi arabicum, Polyvinylalkohol, Polyvinylacetat.

Es können Farbstoffe, wie anorganische Pigmente, z.B. Eisenoxid, Titanoxid, Ferrocyanblau und organische Farbstoffe, wie Alizarin- Phthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gew.% Wirkstoff, vorzugsweise zwischen 0,5 und 90%.

Die Wirkstoffe können als solche, in form ihrer Formulierungen oder den daraus durch weiters Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Gieben, Tauchen, Spritzen, Sprühen, Vernebeln, Verdampfen, Injizieren, Verschlämmen, Stäuben, Streuen, Trockenbeizen, Feuchtbeizen, Nassbeizen, Schlämmbeizen oder Inkrustieren.

Bei der Behandlung von Pflanzenteilen können die Wirkstoffkonzentrationen in den Anwendungsformen in einem größeren Bereich veriiert werden. Sie liegen im allgemeinen zwischen 1 und 0,0001 Gew%, vorzugsweise zwischen 0,5 und 0,001%.

Bei der Anwendung der Wirkstoff zur Behandlung von Pilzinfektionen liegen die Aufwandmengen zwischen 0,015, und 4 kg Wirkstoff/ha Fläche.

Zum Oberflächenschutz von Bäumen und Früchten können die Wirkstoffe auch in Verbindung mit Kunststoffdispersionen 0,25%ig bis 5%ig, bezogen auf das Gewicht der Dispersion, verwendet werden.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.%, vorzugsweise 0,0001 bis 0,02%, am Wirkungsort erforderlich.

## Ausführungsbeispiele

### Beispiel 1

5,07 g (0,02 mol) 4-Methyl-3-phenyl-6-(piperidino-1)-pyridazin wurden in 30 ml Aceton gelöst und 3,12 g (0,022 mol) Methyliodid zugefügt und die Mischung 23 Stunden bei Raumtemperatur belassen. Anschließend wurde das Reaktionsgemisch in Vakuum zur Trockene gebracht und der anfallende Rückstand mit Methylacetat bei 60°C zur Kristallisation gebracht. Die anfallenden Kristalle wurden mit Methylacetate und Ether gewaschen und zur Gewichtskonstanz getrocknet. Es wurden, 7,6 g (0,019 mol) 2(1),4-Dimethyl-3-phenyl-6-(piperidino-1)-pyridaziniumiodid mit Fp.169—171°C erhalten.

### Beispiel 2

5,1 g (0.02 mol) 3-Phenyl-6-(4′-methylpiperidino-1)-pyridazin wurden mit 30 ml (0,2 mol) 1-Iodhexan versetzt und die Mischung 6 Stunden bei 100°C gerührt. Anschließend wurde die Reaktionsmischung in Ether eingerührt und auf −30°C gekühlt. Der anfallende Feststoff wurde isoliert, mit Ether gewaschen und zur Gewichtskonstanz getrocknet. Es wurden 8,92 g (0,019 mol); 2,(1)Hexyl-3-phenyl-6-(4′-methylpiperidino-1)-pyridaziniumiodid mit Fp. 63—73°C erhalten.

### Beispiel 3

6,0 g (0,024 mol) 4-Methyl-3-phenyl-6-(piperidino-1)-pyridazin wurden in 100 ml Aceton gelöst und 10 ml (0,07 mol) p-Toluolsulfonsäuremethylester zugefügt und die Mischung auf Rückflußtemperatur gehalten. Dann wurde das Lösungsmittel abgedampft und der verbleibende Rückstand mit methylacetat zur Kristallisation gebracht. Es wurden 10 g (0,023 mol) 2(1),4-Dimethyl-3-phenyl-6-(piperidino-1)-pyridaziniumtosylat mit Fp. 135—141°C erhalten.

### Beispiel 4

6,4 g (0,024 mol) Silbersalycylat. $H_2O$ wurden in 5 l Wasser gelöst und bei Zimmertemperatur zu 300 ml einer wässrigen Lösung von 10 g (0,025 mol) 2,4-Dimethyl-3-phenyl-6(4′-methylpiperidino-1)-pyridaziniumiodid zugefügt. Das sofort als Niederschlag anfallende Silberiodid wurde abgetrennt und die erhaltene Lösung bei vermindertem Druck und bei einer Temperatur von 60°C vom Wasser befreit. Es wurden 10,1 g (0,024 mol) an 2,4-Dimethyl-3-phenyl-6-(4′-methylpiperidino-1)-pyridaziniumsalycclat im Form eines Ols erhalten.

### Beispiel 5

6,0 g (0,024 mol) 4-Methyl-3-phenyl-6-(piperidino-1)-pyridazin wuden in 150 ml Aceton gelöst. Nach Zugabe von 40 g (0,42 mol) Methylbromid wurde die Mischung 20 Stunden bei 80°C unter einem Druck von 5 bar gerührt. Nach Abziehen der flüchtigen Bestandteile wurde der Rückstand mit Methylacetat zur Kristallisation gebracht. Es wurden 7,7 g (0,022 mol) 2 (1), 4-Dimethyl-3-phenyl-6-(piperidino-1)-pyridaziniumbromid mit Fp. 185—197°C erhalten.

Auf analoge Weise wurden die in der Tabelle zusammengefaßten Verbindungen erhalten.

Die als Ausgangsverbindungen für die Quartärsalze verwendeten Pyridazinbasen sind nicht mit einer Beispielnummer versehen und wei die Quartärsalze durch ihren Schmelzpunkt charakterisiert(*. . . . . . Zersetzung).

In der Tabelle bedeuten:

| | | | |
|---|---|---|---|
| Me | Methyl | MePip | Methylpiperidino |
| Et | Ethyl | OHPip | Hydroxypiperidino |
| iPr | i-Propyl | DMePip | Dimethylpiperidino |
| nPr | n-Propyl | OHFtPip | 4-(1-Hydroxyethyl-2)-piperidino |
| iBu | i-Butyl | OHMePip | Hydroxymethylpiperidino |
| nBu | n-Butyl | Bz | Benzyl |
| sBu | sec-Butyl | BzPip | 4-Benzylpiperidino |
| tBu | t-Butyl | DClBZ | 2,4-Dichlorbenzyl |
| Bul | 2-Butenyl | Mor | Morpholino |
| nPe | n-Pentyl | DMeMor | 2,6-Dimethylmorpholino |
| nHex | n-Hexyl | Aza | Azocycloheptyl |
| nHep | n-Heptyl | PyPaz | 4-(-2-pyridyl)-1-piperazino |
| nOct | n-Octyl | Phac | Phenacyl |
| Ocde | Octadecyl | Acty | Acetonyl |
| All | Allyl | Ac | Acetat |
| OHEt | 2-Hydroxyethyl | TFAc | Trifluoracetat |
| Moxet | Methoxyethyl | Lac | Lactat |
| ClOHPr | 3-Chlor-2-hydroxypropyl-1 | Cit | Citrat |
| CNMe | Cyanomethyl | Bzat | Benzoat |
| Ph | Phenyl | H | Wasserstoff |
| ClPh | Chlorphenyl | I | Iod |
| DClPh | Dichlorphenyl | Br | Brom |
| MePh | 4-Methylphenyl | Cl | Chlor |
| TMoxPh | Trimethoxyphenyl | Mos | Methosulfat |
| MoxPh | 4-Methoxyphenyl | Nit | Nitrat |
| PhOEt | 2-Phenoxyethyl | Hst | Hydrogensulfat |
| PhPr | 3-Phenylpropyl | Mso | Methylsulfonat |
| DOHPr | 2,3-Dihydroxypropyl | TFMso | Trifluormethansulfonat |
| POP | 3-Phenoxy-2-hydroxypropyl | IPrPh | p-i-Propylphenyl |
| ClPOP- | 3-(2'-Chlorphenoxy)-2-hydroxy-1-propyl | nBuPip | 4-Butylpiperidino |
| DMOB | 3,3-Dimethyl-2-oxobutyl | BoxPip | 4-Butoxypiperidino |
| MCM | Methoxycarbonylmethyl | BuPh | 4-Butylphenyl |
| Pyrr | Pyrrolidino | Hede | Hexadecyl |
| Pip | Piperidino | Pyraz | Pyrazolyl-1 |

| Nr | $R_1$ | $R_2$ | $R_3$ | $R_4$ | $R_5$ | A | Fp°C |
|---|---|---|---|---|---|---|---|
| Base | H | H | Ph | H | - | - | 149-153 |
| 6 | H | H | Ph | H | nBu | I | 257-259* |
| Base | H | nBu | Ph | H | - | - | 136-138 |
| 7 | H | nBu | Ph | H | Me | I | 235-238 |
| 8 | H | nBu | Ph | H | Et | I | 202-204 |
| 9 | H | nBu | Ph | H | iPr | I | 85-88,161 |
| 10 | H | nBu | Ph | H | All | Br | 148-158,208-210,5 |
| 11 | H | nBu | Ph | H | nBu | I | 189-192 |
| Base | H | Moxet | Ph | H | - | - | 91-96 |
| 12 | H | Moxet | Ph | H | Me | I | 120-127,173 |
| 13 | H | Moxet | Ph | H | Et | I | 100-106,171 |
| 14 | H | Moxet | Ph | H | nBu | I | 150-150,5 |
| Base | Me | nBu | Ph | Me | - | - | Öl |
| 15 | Me | nBu | Ph | Me | Me | I | 153-156 |
| 16 | Me | nBu | Ph | Me | Et | I | 106-109 |
| 17 | Me | nBu | Ph | Me | All | Br | 70-80 |
| Base | Me | nBu | Ph | H | - | - | 61-65 |
| 18 | Me | nBu | Ph | H | Me | I | |
| 19 | Me | nBu | Ph | H | Et | I | |
| 20 | Me | nBu | Ph | H | iPr | I | |
| 21 | Me | nBu | Ph | H | nBu | I | |
| Base | Et | Et | Ph | H | - | - | 66-69 |
| 22 | Et | Et | Ph | H | Me | I | 167-169 |
| 23 | Et | Et | Ph | H | Et | I | 167-168 |
| 24 | Et | Et | Ph | H | iPr | I | 165-196* |
| 25 | Et | Et | Ph | H | All | Br | 100-103 |
| 26 | Et | Et | Ph | H | nBu | I | |
| Base | All | All | Ph | H | - | - | 58-61,5 |
| 27 | All | All | Ph | H | Me | I | |
| Base | Me | OHEt | Ph | Me | - | - | 134-136 |
| 28 | Me | OHEt | Ph | Me | Me | I | 113-117 |
| 29 | Me | OHEt | Ph | Me | Et | I | 126-128,5 |
| 30 | Me | OHEt | Ph | Me | nBu | I | 137-139 |

| Nr | $R_1$ | $R_2$ | $R_3$ | $R_4$ | $R_5$ | A | Fp°C |
|---|---|---|---|---|---|---|---|
| Base | H | Cyhex | Ph | H | - | - | 173-175 |
| 31 | H | Cyhex | Ph | H | Me | I | 80-95;245-248 |
| Base | Me | Cyhex | Ph | H | - | - | 120-122 |
| 32 | Me | Cyhex | Ph | H | Me | I | 171-178 |
| 33 | Me | Cyhex | Ph | H | Et | I | 145-148 |
| 34 | Me | Cyhex | Ph | H | iPr | I | |
| 35 | Me | Cyhex | Ph | H | nBu | I | |
| Base | Me | Cyhex | Ph | Me | - | - | 75-80 |
| 36 | Me | Cyhex | Ph | Me | Me | I | 190-196* |
| 37 | Me | Cyhex | Ph | Me | All | Br | 161-168* |
| Base | Me | Ph | Ph | H | - | - | 117-121 |
| 38 | Me | Ph | Ph | H | Me | I | 171-178 |
| 39 | Me | Ph | Ph | H | Et | I | 110-122 |
| 40 | Me | Ph | Ph | H | nPr | I | |
| 41 | Me | Ph | Ph | H | iPr | I | 178-187 |
| 42 | Me | Ph | Ph | H | All | Br | |
| 43 | Me | Ph | Ph | H | nBu | I | |
| Base | H | Ph | Ph | Me | - | - | 179-183 |
| 44 | H | Ph | Ph | Me | Me | I | 240-246 |
| Base | H | Ph | Ph | H | - | - | 195-197 |
| 45 | H | Ph | Ph | H | Me | I | 197-215 |
| 46 | H | Ph | Ph | H | Et | I | 189-193 |
| 47 | H | Ph | Ph | H | nPr | I | 190,220 |
| 48 | H | Ph | Ph | H | iPr | I | 150,195-197 |
| 49 | H | Ph | Ph | H | nBu | I | 195,212 |
| 50 | H | Ph | Ph | H | nPe | I | 169,186 |
| 51 | H | Ph | Ph | H | nHep | I | 144,158 |
| 52 | H | Ph | Ph | H | nOct | I | 118,135 |
| Base | H | p-ClPh | Ph | H | - | - | 230-232 |
| 53 | H | p-ClPh | Ph | H | Me | I | 198-215 |
| 54 | H | p-ClPh | Ph | H | Et | I | 194-200 |
| 55 | H | p-ClPh | Ph | H | nPr | I | 204-210 |
| 56 | H | p-ClPh | Ph | H | nBu | I | 182,225 |
| 57 | H | p-ClPh | Ph | H | nPe | I | 168,185 |
| 58 | H | p-ClPh | Ph | H | nHep | I | 166,180 |

| Nr | $R_1$ | $R_2$ | $R_3$ | $R_4$ | $R_5$ | A | Fp°C |
|---|---|---|---|---|---|---|---|
| 59 | H | p-ClPh | Ph | H | nOct | I | 179-190 |
| Base | H | 3,4-DClPh | Ph | H | - | - | 261-263 |
| 60 | H | 3,4-DClPh | Ph | H | Me | I | 209,230 |
| 61 | H | 3,4-DClPh | Ph | H | Et | I | 198,218 |
| 62 | H | 3,4-DClPh | Ph | H | nPr | I | 208,214 |
| 63 | H | 3,4-DClPh | Ph | H | nBu | I | 222,235 |
| 64 | H | 3,4-DClPh | Ph | H | nPe | I | 188,230 |
| 65 | H | 3,4-DClPh | Ph | H | nHep | I | 190,210 |
| 66 | H | 3,4-DClPh | Ph | H | nOct | I | 184,202 |
| Base | H | 2,3-DClPh | Ph | H | - | - | 167-171 |
| 67 | H | 2,3-DClPh | Ph | H | Et | I | 192,235 |
| 68 | H | 2,3-DClPh | Ph | H | nPr | I | 162-171 |
| Base | H | 3,5-DClPh | Ph | H | - | - | 226-229 |
| 69 | H | 3,5-DClPh | Ph | H | Et | I | 213-220 |
| Base | H | 3-ClPh | Ph | H | - | - | 198-200 |
| 70 | H | 3-ClPh | Ph | H | Me | I | 193,210 |
| 71 | H | 3-ClPh | Ph | H | Et | I | 166-178 |
| 72 | H | 3-ClPh | Ph | H | nPr | I | 180-191 |
| 73 | H | 3-ClPh | Ph | H | nBu | I | 160-175 |
| 74 | H | 3-ClPh | Ph | H | nPe | I | 150,165 |
| 75 | H | 3-ClPh | Ph | H | nHep | I | 170,190 |
| 76 | H | 3-ClPh | Ph | H | nOct | I | 152,185 |
| Base | H | 2-ClPh | Ph | H | - | - | 134-137 |
| 77 | H | 2-ClPh | Ph | H | Me | I | 178,195 |
| 78 | H | 2-ClPh | Ph | H | Et | I | 186,207 |
| 79 | H | 2-ClPh | Ph | H | nPr | I | 164-175 |
| 80 | H | 2-ClPh | Ph | H | nBu | I | 133,151 |
| 81 | H | 2-ClPh | Ph | H | nPe | I | 116,132 |
| 82 | H | 2-ClPh | Ph | H | nHep | I | 101,126 |
| 83 | H | 2-ClPh | Ph | H | nOct | I | 100,122 |

| Nr | $R_1NR_2$ | $R_3$ | $R_4$ | $R_5$ | A | Fp°C |
|---|---|---|---|---|---|---|
| Base | Pyrr | Ph | Me | - | - | 143-145 |
| 84 | Pyrr | Ph | Me | Me | I | 160-164 |
| 85 | Pyrr | Ph | Me | Et | I | 184-186 |
| 86 | Pyrr | Ph | Me | iPr | I | 193-198 |
| 87 | Pyrr | Ph | Me | All | Br | 164-170 |
| 88 | Pyrr | Ph | Me | nBu | I | 125-127 |
| 89 | Pyrr | Ph | Me | Phac | Cl | 168-170 |
| 90 | Pyrr | Ph | Me | POP | Cl | 168-183 |
| Base | Pyrr | Ph | H | - | - | 158-160 |
| 91 | Pyrr | Ph | H | Me | I | 107-112* |
| 92 | Pyrr | Ph | H | Et | I | 160-164 |
| 93 | Pyrr | Ph | H | iPr | I | 173-176 |
| 94 | Pyrr | Ph | H | All | Br | 153-160* |
| 95 | Pyrr | Ph | H | nBu | I | 130-140 |
| 96 | Pyrr | Ph | H | Acty | Cl | 156-160 |
| Base | Pip | Ph | H | - | - | 135-136 |
| 97 | Pip | Ph | H | H | I | 201-218 |
| 98 | Pip | Ph | H | Me | I | 117-120 |
| 99 | Pip | Ph | H | Me | Mos | Öl |
| 100 | Pip | Ph | H | Et | Br | 142-150 |
| 101 | Pip | Ph | H | Et | I | 126-129 |
| 102 | Pip | Ph | H | nPr | I | 100-106 |
| 103 | Pip | Ph | H | iPr | I | 150-165 |
| 104 | Pip | Ph | H | All | Br | 117-120* |
| 105 | Pip | Ph | H | nBu | Br | |
| 106 | Pip | Ph | H | nBu | I | 105-107 |
| 107 | Pip | Ph | H | nOct | I | Öl |
| 108 | Pip | Ph | H | Bz | Br | 145-148 |
| 109 | Pip | Ph | H | CNMe | Cl | 183-186* |
| 110 | Pip | Ph | H | Acty | Cl | 162-168 |
| 111 | Pip | Ph | H | DMOB | Br | 152,5-156 |
| Base | Pip | Ph | Me | - | - | 117-118 |
| 112 | Pip | Ph | Me | H | Br | 265-270 |
| 113 | Pip | Ph | Me | Me | Mos | Öl |

| Nr | $R_1NR_2$ | $R_3$ | $R_4$ | $R_5$ | A | Fp°C |
|---|---|---|---|---|---|---|
| 114 | Pip | Ph | Me | Et | I | 145-146 |
| 115 | Pip | Ph | Me | nPr | I | 144-147 |
| 116 | Pip | Ph | Me | iPr | I | 194-198 |
| 117 | Pip | Ph | Me | All | Br | 170-172 |
| 118 | Pip | Ph | Me | All | Cl | 161-170* |
| 119 | Pip | Ph | Me | Bz | Cl | 174-176 |
| 120 | Pip | Ph | Me | MCM | Br | 148-152* |
| 121 | Pip | Ph | Me | CNMe | Cl | 205-220 |
| 122 | Pip | Ph | Me | OHFt | Cl | 231-237 |
| 123 | Pip | Ph | Me | ClOHPr | Cl | 208-212* |
| 124 | Pip | Ph | Me | DOHPr | Cl | 185-203 |
| 125 | Pip | Ph | Me | PhOFt | Br | 179-182 |
| 126 | Pip | Ph | Me | PhPr | Br | Öl |
| 127 | Pip | Ph | Me | ClPOP | Cl | 188-194 |
| Base | Pip | Ph | Et | - | - | 81-83 |
| 128 | Pip | Ph | Et | Me | I | 168-172 |
| 129 | Pip | Ph | Et | Et | I | 138-141 |
| 130 | Pip | Ph | Et | Et | Br | 170-173 |
| 131 | Pip | Ph | Et | nPr | Br | 209-216* |
| 132 | Pip | Ph | Et | nPr | I | 176-180 |
| 133 | Pip | Ph | Et | iPr | I | 199-202* |
| 134 | Pip | Ph | Et | All | Br | 181-188 |
| 135 | Pip | Ph | Et | nBu | I | 134-138 |
| 136 | Pip | Ph | Et | nBu | Br | 180-184 |
| 137 | Pip | Ph | Et | iBu | I | 143-147 |
| 138 | Pip | Ph | Et | nPe | I | 126-129 |
| 139 | Pip | Ph | Et | nHex | I | 90-94 |
| 140 | Pip | Ph | Et | nHep | I | 75-80 |
| 141 | Pip | Ph | Et | nOct | I | Öl |
| Base | 2-MePip | Ph | H | - | - | 99-102 |
| 142 | 2-MePip | Ph | H | Me | I | 125-133 |
| 143 | 2-MePip | Ph | H | Et | I | 142-149 |
| 144 | 2-MePip | Ph | H | iPr | I | 130-149 |
| 145 | 2-MePip | Ph | H | All | Br | 130-136 |
| 146 | 2-MePip | Ph | H | nBu | I | 150-154 |

| Nr | $R_1$ N $R_2$ | $R_3$ | $R_4$ | $R_5$ | A | Fp°C |
|---|---|---|---|---|---|---|
| Base | 2-MePip | Ph | Me | - | - | 98-103 |
| 147 | 2-MePip | Ph | Me | Me | I | 179-184 |
| 148 | 2-MePip | Ph | Me | iPr | I | 228-231 |
| Base | 2-EtPip | Ph | H | - | - | 82-94 |
| 149 | 2-EtPip | Ph | H | Me | I | 131-136 |
| 150 | 2-EtPip | Ph | H | iPr | I | 130-148 |
| Base | 2-EtPip | Ph | Me | - | - | Öl |
| 151 | 2-EtPip | Ph | Me | Me | I | 177-184 |
| 152 | 2-EtPip | Ph | Me | Et | I | 118-127 |
| 153 | 2-EtPip | Ph | Me | iPr | I | 174-184 |
| Base | 3-MePip | Ph | H | - | - | 93-96 |
| 154 | 3-MePip | Ph | H | Me | I | 112-120 |
| 155 | 3-MePip | Ph | H | Et | I | 45-57 |
| 156 | 3-MePip | Ph | H | iPr | I | Öl |
| 157 | 3-MePip | Ph | H | All | Br | |
| 158 | 3-MePip | Ph | H | nBu | I | Öl |
| Base | 3-MePip | Ph | Me | - | - | 113-115 |
| 159 | 3-MePip | Ph | Me | Me | I | 149-152 |
| 160 | 3-MePip | Ph | Me | Et | I | 115-122 |
| 161 | 3-MePip | Ph | Me | nPr | I | 143-149 |
| 162 | 3-MePip | Ph | Me | iPr | I | 183-195 |
| 163 | 3-MePip | Ph | Me | All | Br | 184-191 |
| 164 | 3-MePip | Ph | Me | nBu | I | 177-181 |
| Base | 4-MePip | Ph | H | - | - | 108-110 |
| 165 | 4-MePip | Ph | H | Me | I | 127-132 |
| 166 | 4-MePip | Ph | H | Et | I | 122-125 |
| 167 | 4-MePip | Ph | H | iPr | I | 148-161 |
| 168 | 4-MePip | Ph | H | All | Br | |
| 169 | 4-MePip | Ph | H | nBu | I | 69-74 |
| 170 | 4-MePip | Ph | H | tBu | I | Öl |
| 171 | 4-MePip | Ph | H | iPe | I | Öl |
| 172 | 4-MePip | Ph | H | nHep | I | 94-100 |
| 173 | 4-MePip | Ph | H | nOct | I | 73-82 |
| Base | 4-MePip | Ph | Me | - | - | 117-118 |
| 174 | 4-MePip | Ph | Me | Me | I | 174-176 |

| Nr | $R_1NR_2$ | $R_3$ | $R_4$ | $R_5$ | A | Fp°C |
|---|---|---|---|---|---|---|
| 175 | 4-MePip | Ph | Me | Me | $NO_2$ | 145-149 |
| 176 | 4-MePip | Ph | Me | Me | Mos | |
| 177 | 4-MePip | Ph | Me | Et | I | 144-146 |
| 178 | 4-MePip | Ph | Me | All | Br | 160-162 |
| 179 | 4-MePip | Ph | Me | Bz | Br | 91-97* |
| 180 | 4-MePip | Ph | Me | Me | Nit | 160-165 |
| 181 | 4-MePip | Ph | Me | Me | Hst | 128-131 |
| 182 | 4-MePip | Ph | Me | Me | TFMso | 78-89 |
| 183 | 4-MePip | Ph | Me | Me | Ac | |
| 184 | 4-MePip | Ph | Me | Me | TFAc | |
| 185 | 4-MePip | Ph | Me | Me | Lac | |
| 186 | 4-MePip | Ph | Me | Me | Cit | |
| 187 | 4-MePip | Ph | Me | Me | Bzat | 55-70 |
| Base | 4-MePip | Ph | Et | - | - | 75-80 |
| 188 | 4-MePip | Ph | Et | Me | I | 137-140,169-171 |
| 189 | 4-MePip | Ph | Et | Et | Br | 179-183 |
| 190 | 4-MePip | Ph | Et | Et | I | 102-106 |
| 191 | 4-MePip | Ph | Et | All | Br | 132-136 |
| 192 | 4-MePip | Ph | Et | nPr | Br | 127-131 |
| 193 | 4-MePip | Ph | Et | nPr | I | 138-140 |
| 194 | 4-MePip | Ph | Et | iPr | I | 190-193 |
| 195 | 4-MePip | Ph | Et | nBu | I | 114-118 |
| 196 | 4-MePip | Ph | Et | nBu | Br | 142-149 |
| 197 | 4-MePip | Ph | Et | Bul | Br | 124-128 |
| 198 | 4-MePip | Ph | Et | iBu | I | 154-163 |
| 199 | 4-MePip | Ph | Et | nPe | I | 94-99 |
| 200 | 4-MePip | Ph | Et | nHex | I | 109-113 |
| 201 | 4-MePip | Ph | Et | nHep | I | 66-71 |
| 202 | 4-MePip | Ph | Et | nOct | I | 74-81 |
| 203 | 4-MePip | Ph | Et | nOct | Br | Öl |
| 204 | 4-MePip | Ph | Et | Cyhex | I | 167-172,179-192 |
| Base | 3-OHPip | Ph | H | - | - | 133-135 |
| 205 | 3-OHPip | Ph | H | Me | I | |
| Base | 3-OHPip | Ph | Me | - | - | 136-140 |
| 206 | 3-OHPip | Ph | Me | Me | I | 87-95 |

| Nr | $R_1 N R_2$ | $R_3$ | $R_4$ | $R_5$ | A | Fp°C |
|---|---|---|---|---|---|---|
| Base | 4-OHPip | Ph | H | - | - | 149-152 |
| 207 | 4-OHPip | Ph | H | Me | I | 184-187* |
| Base | 4-OHPip | Ph | Me | - | - | 120-123 |
| 208 | 4-OHPip | Ph | Me | Me | I | 215-221 |
| Base | OHEtPip | Ph | H | - | - | 143-147 |
| 209 | OHEtPip | Ph | H | Me | I | 130-137 |
| Base | OHEtPip | Ph | Me | - | - | 82-85 |
| 210 | OHEtPip | Ph | Me | Me | I | 137-140,170-172 |
| Base | 3,3-DMePip | Ph | H | - | - | 106-108 |
| 211 | 3,3-DMePip | Ph | H | Me | I | 160-175 |
| 212 | 3,3-DMePip | Ph | H | Et | I | 127-137 |
| 213 | 3,3-DMePip | Ph | H | All | Br | 140-142,5 |
| 214 | 3,3-DMePip | Ph | H | nBu | I | 115-120 |
| Base | 3,3-DMePip | Ph | Me | - | - | 115-116 |
| 215 | 3,3-DMePip | Ph | Me | Me | I | 188-192 |
| 216 | 3,3-DMePip | Ph | Me | Et | I | 175-177 |
| 217 | 3,3-DMePip | Ph | Me | All | Br | 165-169 |
| 218 | 3,3-DMePip | Ph | Me | nBu | I | 163-164,5 |
| Base | 3,5-DMePip | Ph | H | - - | - | 88-94 |
| 219 | 3,5-DMePip | Ph | H | Me | I | 169-184 |
| 220 | 3,5-DMePip | Ph | H | Et | I | 147-159 |
| 221 | 3,5-DMePip | Ph | H | iPr | I | 135-181 |
| 222 | 3,5-DMePip | Ph | H | nBu | I | |
| Base | BzPip | Ph | H | - | - | 147-150 |
| 223 | BzPip | Ph | H | Me | I | 192-195 |
| 224 | BzPip | Ph | H | Et | I | 193-198 |
| 225 | BzPip | Ph | H | All | Br | 109-124 |
| 226 | BzPip | Ph | H | nBu | I | 144-146 |
| Base | BzPip | Ph | Me | - | - | 120-124 |
| 227 | BzPip | Ph | Me | Me | I | 196-205 |
| 228 | BzPip | Ph | Me | Et | I | 190-193 |
| 229 | BzPip | Ph | Me | All | Br | 174-177 |
| 230 | BzPip | Ph | Me | nBu | I | 128-133 |
| Base | Aza | Ph | H | - | - | 115-118 |
| 231 | Aza | Ph | H | Me | I | 135-153 |

| Nr | $R_1NR_2$ | $R_3$ | $R_4$ | $R_5$ | A | Fp°C |
|---|---|---|---|---|---|---|
| 232 | Aza | Ph | H | Et | I | 82-88 |
| 233 | Aza | Ph | H | nPr | I | 87-110 |
| 234 | Aza | Ph | H | iPr | I | 173-199 |
| 235 | Aza | Ph | H | All | Br | 120-125 |
| 236 | Aza | Ph | H | nBu | I | 103-105 |
| 237 | Aza | Ph | H | iBu | I | 84-90 |
| 238 | Aza | Ph | H | sBu | I | 150-178 |
| 239 | Aza | Ph | H | nPe | I | |
| 240 | Aza | Ph | H | iPe | I | 108-115 |
| 241 | Aza | Ph | H | nHex | I | |
| 242 | Aza | Ph | H | nHep | I | |
| 243 | Aza | Ph | H | nOct | I | |
| Base | Aza | Ph | Me | - | - | 82-84 |
| 244 | Aza | Ph | Me | Me | I | 173-180 |
| 245 | Aza | Ph | Me | Et | I | 118-129 |
| 246 | Aza | Ph | Me | nPr | I | 103-153 |
| 247 | Aza | Ph | Me | iPr | I | 184-187 |
| 248 | Aza | Ph | Me | All | Br | 150-153 |
| 249 | Aza | Ph | Me | nBu | I | 132-137 |
| Base | Mor | Ph | H | - | - | 155-155,5 |
| 250 | Mor | Ph | H | H | Cl | 165-192 |
| 251 | Mor | Ph | H | Me | I | 190-192* |
| 252 | Mor | Ph | H | Et | I | 125-130 |
| 253 | Mor | Ph | H | iPr | I | 134-139 |
| 254 | Mor | Ph | H | All | Br | 154-159 |
| 255 | Mor | Ph | H | nBu | Br | 145-148 |
| 256 | Mor | Ph | H | nBu | I | 149-152 |
| Base | Mor | Ph | Me | - | - | 121-122 |
| 257 | Mor | Ph | Me | H | Cl | 193-215* |
| 258 | Mor | Ph | Me | H | I | 250-272* |
| 259 | Mor | Ph | Me | Me | I | 220-225 |
| 260 | Mor | Ph | Me | Et | I | 133-137 |
| 261 | Mor | Ph | Me | iPr | I | 215-220* |
| 262 | Mor | Ph | Me | All | Br | 175-179 |
| 263 | Mor | Ph | Me | nBu | I | 175-177 |

| Nr | $R_1 N R_2$ | $R_3$ | $R_4$ | $R_5$ | A | Fp°C |
|---|---|---|---|---|---|---|
| 264 | Mor | Ph | Me | Acty | Cl | 210-215 |
| 265 | Mor | Ph | Me | Bz | Br | 173-178 |
| 266 | Mor | Ph | Me | DClBz | Cl | 143-150 |
| Base | DMeMor | Ph | H | - | - | 104-112 |
| 267 | DMeMor | Ph | H | Me | I | 158-173 |
| 268 | DMeMor | Ph | H | Et | I | 148-152 |
| 269 | DMeMor | Ph | H | iPr | I | 164-197 |
| 270 | DMeMor | Ph | H | All | Br | 160-182 |
| 271 | DMeMor | Ph | H | nBu | I | 126-142 |
| 272 | DMeMor | Ph | H | nOct | I | 115-124 |
| Base | DMeMor | Ph | Me | - | - | 175-180 |
| 273 | DMeMor | Ph | Me | Me | I | 243-250 |
| 274 | DMeMor | Ph | Me | Et | I | 221-229 |
| 275 | DMeMor | Ph | Me | iPr | I | 170-210 |
| 276 | DMeMor | Ph | Me | All | Br | 211-227 |
| 277 | DMeMor | Ph | Me | nBu | I | 224-233 |
| Base | Pypaz | Ph | H | - | - | 197-199 |
| 278 | Pypaz | Ph | H | Et | I | 218-224* |
| Base | Pip | p-ClPh | H | - | - | 176-178 |
| 279 | Pip | p-ClPh | H | H | Br | 227-232* |
| 280 | Pip | p-ClPh | H | Me | I | Öl |
| 281 | Pip | p-ClPh | H | Et | Br | Öl |
| 282 | Pip | p-ClPh | H | Et | I | |
| 283 | Pip | p-ClPh | H | nPr | Br | 81-95 |
| 284 | Pip | p-ClPh | H | All | Br | |
| 285 | Pip | p-ClPh | H | nBu | Br | Öl |
| Base | Pip | p-ClPh | Me | - | - | 141-143 |
| 286 | Pip | p-ClPh | Me | H | Br | 224-230 |
| 287 | Pip | p-ClPh | Me | Me | I | 176-184* |
| Base | Mor | p-ClPh | H | - | - | 194-196 |
| 288 | Mor | p-ClPh | H | H | Br | 248-252 |
| 289 | Mor | p-ClPh | H | Me | I | 159-163* |
| Base | Pip | MePh | H | - | - | 150-151 |
| 290 | Pip | MePh | H | H | Br | 207-211 |
| 291 | Pip | MePh | H | H | Cl | 162-165 |

| Nr | $R_1NR_2$ | $R_3$ | $R_4$ | $R_5$ | A | Fp°C |
|---|---|---|---|---|---|---|
| 292 | Pip | MePh | H | Me | I | Öl |
| 293 | Pip | MePh | H | All | Br | |
| Base | 4-MePip | MePh | H | - | - | 164-167 |
| 294 | 4-MePip | MePh | H | H | Br | 251-254 |
| 295 | 4-MePip | MePh | H | H | Cl | 177-187* |
| 296 | 4-MePip | MePh | H | Me | I | 173-181* |
| 297 | 4-MePip | MePh | H | All | Br | Öl |
| 298 | 4-MePip | MePh | H | BuI | Br | Öl |
| Base | Pip | 3,4,5-TMoxPh | H | - | - | 170,5-172 |
| 299 | Pip | 3,4,5-TMoxPh | H | Me | I | 155-164 |
| 300 | Pip | 3,4,5-TMoxPh | H | Et | I | 127-134 |
| 301 | Pip | 3,4,5-TMoxPh | H | All | Br | 97-110 |
| Base | Pip | tBu | H | - | - | 106-108 |
| 302 | Pip | tBu | H | Me | I | 146-150 |
| 303 | Pip | tBu | H | Et | Br | 92-101 |
| 304 | Pip | tBu | H | Et | I | 141-146 |
| 305 | Pip | tBu | H | nPr | I | 110-122 |
| 306 | Pip | tBu | H | iPr | I | 195-207 |
| 307 | Pip | tBu | H | nBu | I | 106-119 |
| 308 | Pip | tBu | H | nPe | I | 112-122 |
| 309 | Pip | tBu | H | nHep | I | 94-101 |
| 310 | Pip | tBu | H | nOct | I | 94-98 |
| Base | 4-MePip | tBu | H | - | - | 119-122 |
| 311 | 4-MePip | tBu | H | Me | I | 110-119 |
| 312 | 4-MePip | tBu | H | All | Br | Öl |
| 313 | 4-MePip | tBu | H | BuI | Br | 150-157 |
| Base | 3-OHMePip | Ph | H | - | - | 114-116 |
| 314 | 3-OHMePip | Ph | H | Me | I | |
| Base | 3-OHMePip | Ph | Me | - | - | 60-70 |
| 315 | 3-OHMePip | Ph | Me | Me | I | |
| Base | 2-OHMePip | Ph | H | - | - | 117-118,5 |
| 316 | 2-OHMePip | Ph | H | Me | I | |
| 317 | Pip | Ph | Me | MoxEt | Br | 128-131 |
| 318 | 4-MePip | Ph | Me | Me | F | 50-60 |
| 319 | 4-MePip | MePh | H | Et | I | 168,5-170,5 |

| Nr | $R_1NR_2$ | $R_3$ | $R_4$ | $R_5$ | A | Fp°C |
|---|---|---|---|---|---|---|
| 320 | 4-MePip | MePh | H | nPr | I | 158-160* |
| 321 | 4-MePip | MePh | H | iPr | I | 157-173* |
| 322 | 4-MePip | Ph | Me | Me | $ClO_4$ | 118,5-121 |
| 323 | 4-MePip | MePh | H | nBu | I | 127-138 |
| 324 | 4-OHPip | Ph | H | Et | I | 161-170 |
| 325 | 4-OHPip | Ph | H | iPr | I | 175-207* |
| 326 | 4-OHPip | Ph | H | nBu | I | 132-137 |
| 327 | 4-OHPip | Ph | Me | nBu | I | 183-185,5 |
| 328 | 3-OHPip | Ph | H | Et | I | Öl |
| 329 | 3-OHPip | Ph | H | nBu | I | 136-142 |
| 330 | 3-OHPip | Ph | Me | Et | I | 182-185 |
| 331 | 3-OHPip | Ph | Me | nBu | I | 141-145 |
| 332 | OHEtPip | Ph | H | Et | I | 88-99 |
| 333 | OHEtPip | Ph | H | nBu | I | 82-97 |
| 334 | OHEtPip | Ph | Me | Et | I | 102-110 |
| 335 | OHEtPip | Ph | Me | nBu | I | 91-98* |
| 336 | 3-OHMePip | Ph | H | Et | I | Öl |
| 337 | 3-OHMePip | Ph | H | nBu | I | Öl |
| 338 | 2-OHMePip | Ph | H | nBu | I | Öl |
| 339 | Pip | 2,3,4-TMoxPh | H | Me | I | 143,5-146 |
| 340 | Pip | 2,3,4-TMoxPh | H | Et | I | 141-143 |
| 341 | Pip | 2,3,4-TMoxPh | H | nPr | I | 129-131,5 |
| 342 | Pip | 2,3,4-TMoxPh | H | iPr | I | 141-156 |
| 343 | Pip | 2,3,4-TMoxPh | H | nBu | I | Öl |
| 344 | Pip | 2,3,4-TMoxPh | H | All | Br | Öl |
| 345 | Pip | 4-MoxPh | H | Me | I | 165-170 |
| 346 | Pip | 4-MoxPh | H | Et | I | 111-117 |
| 347 | Pip | 4-MoxPh | H | nPr | I | 105-113 |
| 348 | Pip | 4-MoxPh | H | iPr | I | 193-197 |
| 349 | Pip | 4-MoxPh | H | nBu | I | 115-126 |
| 350 | Pip | 4-MoxPh | H | iBu | I | 187-196 |
| 351 | Pip | 4-MoxPh | H | All | Br | 151-155 |
| 352 | 4-BzPip | Ph | Me | nPr | Br | 179-181 |
| 353 | 4-BzPip | Ph | Me | nPr | I | 180-182 |
| 354 | 4-BzPip | Ph | Me | iPr | I | 190-191 |

| Nr | $R_1NR_2$ | $R_3$ | $R_4$ | $R_5$ | A | Fp°C |
|---|---|---|---|---|---|---|
| 355 | 4-BzPip | Ph | H | nPr | I | 119-125 |
| 356 | 4-BzPip | Ph | H | iPr | I | 147-152 |
| 357 | 4-BzPip | Ph | H | nPr | Br | 119-122 |
| 358 | Pip | Ph | H | Ocde | I | 56,5-61 |
| 359 | 4-MePip | Ph | H | Ocde | I | 64-67,5 |
| 360 | 4-MePip | Ph | Me | Me | Mso | |
| 361 | 4-MePip | Ph | Me | Et | Br | 204-208* |
| 362 | 4-MePip | Ph | Me | nPr | Br | 151-154 |
| 363 | 4-MePip | Ph | Me | nBu | Br | 152-155 |
| 364 | 4-MePip | Ph | Me | nBu | I | 114-118 |
| 365 | 4-MePip | Ph | Me | BuI | Br | 130-143 |
| 366 | 4-MePip | Ph | Me | nPe | I | 117-121 |
| 367 | 4-MePip | Ph | Me | nHex | I | Öl |
| 368 | 4-MePip | Ph | Me | nHep | I | Öl |
| 369 | 4-MePip | Ph | Me | nOct | I | Öl |
| 370 | 3-MePip | Ph | Me | Et | Br | 170-173 |
| 371 | 3-MePip | Ph | Me | nPr | Br | 185-188 |
| 372 | 3-MePip | Ph | Me | nBu | Br | 188-195 |
| 373 | 3-MePip | Ph | Me | iBu | I | 165-170 |
| 374 | 3-MePip | Ph | Me | BuI | Br | 160-165 |
| 375 | 3-MePip | Ph | Me | nPe | Br | 132-137 |
| 376 | 3-MePip | Ph | Me | nPe | I | 155-158 |
| 377 | 3-MePip | Ph | Me | nHex | Br | 132-135 |
| 378 | 3-MePip | Ph | Me | nHex | I | 119-121 |
| 379 | 3-MePip | Ph | Me | nHep | I | 116-119 |
| 380 | 3-MePip | Ph | Me | nOct | Br | 117-120 |
| 381 | 3-MePip | Ph | Me | nOct | I | 84-88 |
| 382 | Pip | Ph | Me | Et | Br | 200-203 |
| 383 | Pip | Ph | Me | nPr | Br | 188-192 |
| 384 | Pip | Ph | Me | nBu | Br | 191-196 |
| 385 | Pip | Ph | Me | nBu | I | 135-138 |
| 386 | Pip | Ph | Me | iBu | I | 151-155 |
| 387 | Pip | Ph | Me | nPe | Br | 149-152 |
| 388 | Pip | Ph | Me | nPe | I | 129-133 |
| 389 | Pip | Ph | Me | nHex | I | 90-94 |

| Nr | $R_1NR_2$ | $R_3$ | $R_4$ | $R_5$ | A | Fp°C |
|---|---|---|---|---|---|---|
| 390 | Pip | Ph | Me | nHep | I | 83-87 |
| 391 | Pip | Ph | Me | nOct | I | Öl |
| 392 | 4-MePip | Ph | Et | Ocde | I | 71-73,5 |
| Base | 4-MePip | Ph | nBu | - | - | Öl |
| 393 | 4-MePip | Ph | nBu | Me | I | 165-169 |
| 394 | 4-MePip | Ph | nBu | Et | I | 154-157 |
| 395 | 4-MePip | Ph | nBu | nBu | I | 125-129 |
| 396 | 4-MePip | Ph | nBu | nOct | I | 83-87 |
| Base | Aza | Ph | Et | - | - | 80-82 |
| 397 | Aza | Ph | Et | Me | I | 190-194 |
| 398 | Aza | Ph | Et | Et | Br | |
| 399 | Aza | Ph | Et | Et | I | 142-145 |
| 400 | Aza | Ph | Et | All | Br | 126-132 |
| 401 | Aza | Ph | Et | nPr | Br | 121-124 |
| 402 | Aza | Ph | Et | nPr | I | 129-133 |
| 403 | Aza | Ph | Et | iPr | I | 199-201 |
| 404 | Aza | Ph | Et | nBu | Br | 123-128 |
| 405 | Aza | Ph | Et | nBu | I | 120-123 |
| 406 | Aza | Ph | Et | iBu | I | 117-127 |
| 407 | Aza | Ph | Et | nPe | Br | |
| 408 | Aza | Ph | Et | nPe | I | 105-108 |
| 409 | Aza | Ph | Et | nHex | Br | |
| 410 | Aza | Ph | Et | nHex | I | 56-62 |
| 411 | Aza | Ph | Et | nHep | I | 64-68 |
| 412 | Aza | Ph | Et | nOct | Br | Öl |
| 413 | Aza | Ph | Et | nOct | I | |
| Base | Pip | iPrPh | H | - | - | 138-139,5 |
| 414 | Pip | iPrPh | H | Me | I | 148-155 |
| 415 | Pip | iPrPh | H | Et | I | 140-146 |
| 416 | Pip | iPrPh | H | nBu | I | 82-85,90 |
| Base | 4-MePip | iPrPh | H | - | - | 137-138 |
| 417 | 4-MePip | iPrPh | H | Me | I | 128-132 |
| 418 | 4-MePip | iPrPh | H | Et | I | 149-154 |
| 419 | 4-MePip | iPrPh | H | nBu | I | 92-96 |
| 420 | 4-MePip | iPrPh | H | nOct | I | Öl |

| Nr | $R_1$ N $R_2$ | $R_3$ | $R_4$ | $R_5$ | A | Fp°C |
|---|---|---|---|---|---|---|
| Base | Pip | nBuPh | H | - | - | 110-111 |
| 421 | Pip | nBuPh | H | Me | I | 94-104 |
| 422 | Pip | nBuPh | H | nBu | I | |
| Base | 4-MePip | nBuPh | H | - | - | 123-125 |
| 423 | 4-MePip | nBuPh | H | Me | I | 95-101 |
| 424 | 4-MePip | Ph | Et | Dode | Br | 53-56 |
| Base | Pip | BoxPh | H | - | - | |
| 425 | Pip | BoxPh | H | Me | I | |
| Base | Pyraz | Ph | H | - | - | 173-174 |
| 426 | Pyraz | Ph | H | Me | I | 213,5-215* |
| Base | Pyraz | Ph | Me | - | - | 90-92 |
| 427 | Pyraz | Ph | Me | Me | I | 197-199 |

| Nr | $R_1$ | $R_2$ | $R_3$ | $R_4$ | $R_5$ | A | Fp°C |
|---|---|---|---|---|---|---|---|
| 428 | H | Cyhex | Ph | H | Et | I | 75-90;242-246 |
| 429 | H | Cyhex | Ph | H | nBu | I | 172-181;235-240 |
| 430 | H | Ph | Ph | Me | Et | I | 237-243* |
| 431 | H | Ph | Ph | Me | nBu | I | 237-244* |
| 432 | H | Ph | Ph | Me | All | Br | 219-225 |

Beispiel 433

1 Teil 2,4-Dimethyl-6-(4-methylpiperidino-1)-3-phenylpyridaziniumjodid wurden in 3 Teilen N-Methylpyrrolidon gelöst, und mit 1 Teil ethoxyliertem Nonylphenol mit einem durchschnittlichen Gehalt von 5—6 Mol Ethylenoxid/Mol Nonylphenol versetzt. Das erhaltene Konzentrat wurde mit Wasser zu einer Spritzbrühe der gewünschten Konzentration verdünnt.

Beispiel 434

1 Teil 2,4-Dimethyl-6-(4-methylpiperidino-1)-3-phenylpyridaziniumjodid wurden mit 2,5 Teilen Kaolin, 1 Teil Attaclay und 0,5 Teilen Natrium-oleoylmethyltaurid innig vermengt und dann mit einer Planetenkugelmühle 1 Stunde vermahlen. Man erhielt ein benetzbares Pulver, welches leicht in Wasser zu einer Spritzbrühe der gewünschten Konzentration dispergiert werden konnte.

Beispiel 435

1 Teil 2(1)-Butyl-3-phenyl-6-(piperidino-1)-pyridaziniumjodid wurden in 3 Teilen N-Methylpyrrolidon gelöst und 1 Teil ethoxyliertes Nonylphenol mit einem durchschnittlichen Gehalt von 5—6 Molen Ethylenoxid/Mol Nonylphenol zugegeben. Das erhaltene Konzentrat konnte mit Wasser zu einer Spritzbrühe der gewünschten Konzentration verdünnt werden.

Beispiel 436

5 Teile 2(1)-Butyl-3-phenyl-6-(piperidino-1)-pyridaziniumjodid wurden mit 1-Teil hochdisperser gefällter Kieselsäure, 3 Teilen Ataclay, 0,5 Teilen Natriumligninsulfonat und 0,5 Teilen Naoleoylmethyltaurid innig vermengt und anschließend auf einer Planetenkugelmühle 1 Stunde lang vermahlen. Man erhielt ein benetzbares Pulver, welches leicht in Wasser zu einer Spritzbrühe der gewünschten Konzentration dispergiert werden konnte.

Beispiel A

Prophylaktische Wirkung der Verbindungen gegen Echten Mehltau (Frysiphe cichoracearum) auf Gurken, durch Behandlung der Blätter.

10 Tage alte Gurkenpflanzen wurden mit einer wäßrigen Verdünnung des formulierten Wirkstoffes besprüht. Nach dem Trocknen der Spritzflüssigkeit wurden die Pflanzen mit von infizierten Pflanzen gewonnenen Sporen inokuliert. Die Gurkenpflanzen blieben anschließend im Gewächshaus bei rund 60% Luftfeuchtigkeit stehen.

14 Tage nach der künstlichen Infektion wurde der Befall mit Erysiphe cichoracearum nach folgenden Boniturschema ausgewertet:

1 = kein Befall
2 = bis 2,5% der Blattfläche befallen
3 = bis 5,0% der Blattfläche befallen
4 = 5—10% der Blattfläche befallen
5 = 10—15% der Blattfläche befallen
6 = 15—25% der Blattfläche befallen
7 = 25—35% der Blattfläche befallen
8 = 35—67% der Blattfläche befallen
9 = 67% der Blattfläche befallen

Die Wirkstoffe, die Wirkstoffkonzentrationen und die Boniturwerte sind aus der nachfolgenden Tabelle ersichtlich.

| | Wirkstoffkonzentrationen | | | |
|---|---|---|---|---|
| Beispiel Nr. | 100 ppm | 50 ppm | 25 ppm | 10 ppm |
| 287 | 1 | 3 | - | 7 |
| 296 | 1 | 3 | - | - |
| 103 | 1 | 2 | - | - |
| 143 | 1 | 1 | 4 | - |
| 156 | 1 | 2 | 4 | - |
| 166 | 1 | 1 | 4 | 7 |
| 167 | 1 | 1 | 4 | 7 |
| 224 | 1 | 1 | 2 | 4 |
| 152 | 1 | 2 | 5 | — |

Beispiel B

Prophylaktische Wirkung der Verbindungen gegen Getreidemehltau (Erysiphe graminis) auf Weizen und Gerte durch Behandlung der Blätter:

Etwa 10 cm große Weizen-bzw. Gerstenpflanzen wurden mit einer wäßrigen Verdünnung des formulierten Wirkstoffes allseits besprüht. Nach 48 Stunden wurden die behandelten Pflanzen mit von infizierten Pflanzen gewonnenen Conidiosporen des Pilzes Erysiphe graminis var. tritici (a) bzw. Erysiphe graminis var. hordei (b) bestäubt.

Bei rund 22°C blieben die infizierten Pflanzen im Gewächshaus stehen. 10 Tage nach der künstlichen Infektion wurde nach dem im Beispiel 1 beschriebenen Boniturschema ausgewertet.

Die Wirkstoffe, die Wirkstoffkonzentrationen und die Boniturwerte sind aus der nachfolgenden Tabelle ersichtlich:

| | Wirkstoffkonzentrationen | | | | | | | |
|---|---|---|---|---|---|---|---|---|
| Beispiel | 50 ppm | | 25 ppm | | 10 ppm | | 5 ppm | |
| Nr. | (a) | (b) | (a) | (b) | (a) | (b) | (a) | (b) |
| 285 | 2 | 1 | 4 | 1 | 8 | 1 | - | 7 |
| 95 | 1 | 1 | 2 | 1 | 5 | 1 | - | 7 |
| 100 | 1 | 1 | 1 | 1 | 1 | 1 | 3 | 1 |
| 101 | 1 | 1 | 1 | 1 | 1 | 1 | 3 | 1 |
| 106 | 1 | 1 | 1 | 1 | 1 | 1 | 2 | 1 |
| 167 | 1 | 1 | 1 | 1 | 2 | 2 | 4 | 4 |
| 224 | 1 | 1 | 1 | 1 | 3 | 1 | 6 | 1 |
| 236 | 1 | 1 | 1 | 1 | 1 | 2 | - | - |
| 305 | 1 | 1 | 1 | 1 | 5 | 1 | 7 | 6 |
| 163 | 1 | 1 | 1 | 1 | 1 | 1 | 5 | 4 |
| 178 | 1 | 1 | 1 | 1 | 1 | 1 | 4 | 1 |

Beispiel C

Prophylaktische Wirkung der Verbindungen gegen Echten Mehltau (Phodosphaera leucotricha) an Apfelsämlingen:

Junge Apfelsämlinge wurden mit einer wäßrigen Verdünnung des formulierten Wirkstoffes allseits besprüht und blieben anschließend für 24 Stunden im Gewächshaus stehen.

Mit von erkrankten Apfelsämlingen gewonnenen Conidiosporen wurden die behandelten Pflanzen anschließend inokuliert. Dabei konnten beispielsweise die Verbindung Nr. 106, 100, 101, 224 und 178 in einer Wirkstoffkonzentration von 50 ppm den Ausbruch der Krankheit vollständig verhindern.

Beispiel D

Prophylaktische Wirkung gegen Weizenbraunrost (Puccinia recondita) an Weizen:

Zu Prüfung auf protektive Wirksamkeit wurden junge Weizenpflanzen mit einer wäßrigen Sporensuspension von Puccinia recondita inokuliert.

Nach dem Austrocknen wurden die Pflanzen mit der Wirkstoffzubereitung bis zur Tropfnässe besprüht und anschließend wurden die Pflanzen für 24 Stunden bei 20°C und rund 95% Luftfeuchtigkeit in einem Klimaschrank inokuliert.

Bis zum Ausbruch der Krankheit an den Kontrollpflanzen standen die Testpflanzen im Gewächshaus bei rund 20°C und 70% Luftfeuchtigkeit.

In dieser Prüfung verhinderten beispielsweise die Verbindungen Nr. 177, 179, 218, 224 und 106 in einer Konzentration von 100 ppm vollständig den Befall der Pflanzen.

Beispiel E

Fungizide Wirksamkeit im Agar-Plattentest:

Die in Aceton gelösten Wirkstoffe wurden in Bestimmter Konzentration einem verflüssigten Nährboden zugesetzt.

Der Agar wurde in Petrischalen ausgegossen und nach dem Erkalten mit dem Testorganismuns beimpft. Sobald die Kontrolle vom Testorganismus bewachsen war, wurde ausgewertet.

In diesen Prüfungen wurde beispielsweise in einer Konzentration von 400 ppm das Wachstum von Poria placenta und Merulius lacrimans von den Verbindungen Nr 37, 84, 85 und 235 verhindert. Ebenfalls in einer Konzentration von 400 ppm verhinderten die Verbindungen Nr. 214, 223, 224, 225, 226, 283 und 88 das Wachstum von Pseudocercosporella herpotrichoides vollständig. Mit 1000 ppm wurde das Wachstum von Rhizoctonia solani von dem Verbindungen Nr. 177, 178, 163 und 106 unterbunden.

Beispiel F

Bakterizide Wirksamkeit im Agar-Diffusionstest

Von den Wirkstofformulierungen wurden wäßrige Dispersionen hergestellt. Filterpapierplättchen wurden darin getränkt und anschließend auf den beimpften Nährboden gelegt.

Sobald die Kontrolle vom Testorganismus bewachsen war, wurde ausgewertet.

Es verhinderten dabei beispielsweise die Verbindungen Nr. 37, 163, 177, 101 und 106 in einer Konzentration von 1000 ppm das Wachstum von Xanthomonas ssp. rund um das Filterpapierplättchen.

Beispiel G

Finfluß der Wirkstoffe auf das Wachstum der Alge Chlorella fusca:

Zu 70 ml Algensuspension, entsprechend 0,1 mg Algen/1 ml Nährlösung, wurde die zu prüfende Substanz, gelöst in Aceton, zugegeben. Die Algensuspensionen wurden 48 Stunden lang bei künstlicher Beleuchtung und Belüftung gehalten.

Ausgewertet wurde der Prozentwert (P) = Algenkonzentration (in mg) in der Testlösung im Verhältnis zur Kontrolle (= 100 %).

In einer Konzentration von 10 ppm war beispielsweise bei den Verbindungen Nr, 38, 280, 284, 263, 231, 232, 236, 179, 168, 103, 85, 87, und 93 P<1%.

Beispiel H

Wirksamkeit gegen Echten Weizenmehltau der Verbindungen bei Anwendung als Beizmittel:

Je 10 g gereinigtes Saatgut der Winterweizensorte "Gambrinus" wurden sorgfältig mit je 60 g Wirkstoff/100 kg Saatgut gebeizt und nach 24 Stunden eingelegt. Nachdem die Pflanzen das 3-Blattstadium erreicht hatten, wurden die Pflanzen täglich inokuliert. Sobald an den Kontrollpflanzen die Krankheit ausgebrochen war, wurde nach der in Beispiel 1 beschriebenen Methode ausgewertet.

Eine 100 %ige Wirkung bei Bekämpfung des Weizenmehltaues (Erysphe graminus var tritici) erreichten dabei die Verbindungen Nr. 105, 100, 159, 174, 177, 16 und 106.

**Patentansprüche**

1. Pyridaziniumderivate der allgemeinen Formel I

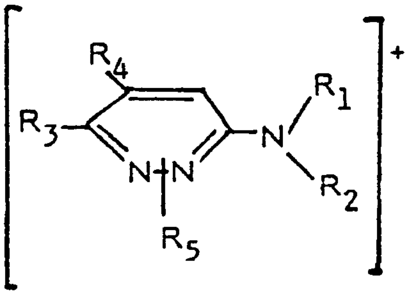

A⁻ (I)

in der

$R_1$ ein Wasserstoffatom, einen gesättigten oder ungesättigten Alkylrest mit 1—4 C-Atomen,

$R_2$ ein Wasserstoffatom, einen geradkettigen oder verzweigten, gesättigten oder ungesättigten Alkylrest mit 1—10 C-Atomen, einen Cyclohexylrest, einen Alkoxyalkylrest mit 1—4 C-Atomen in der Alkylkette, einen Hydroxyalkylrest mit 1—4 C-Atomen in der Alkylkette, einen gegebenenfalls ein- oder mehrfach durch Halogenatome substituierten Phenylrest oder

$R_1$ und $R_2$ gemeinsam mit dem N-Atom einen 5—7 gliedrigen Ring bilden, der als weiteres Heteroatom Stickstoff oder Sauerstoff enthalten kann und gegebenenfalls durch Alkyl-, Hydroxyalkyl-, Phenylalkylreste mit 1—4 C-Atomen in der Alkylkette, einen Hydroxyrest oder einen Pyridylrest substituiert ist mit der Maßgabe, daß der Substituent nicht Hydroxy ist, wenn $R_1$ und $R_2$ gemeinsam mit dem N-Atom einen 6 gliedrigen Ring bilden,

$R_3$ einen gegebenenfalls ein- oder mehrfach durch Halogenatome in 3-, 4-oder 5-Stellung, oder gegebenenfalls ein oder mehrfach durch geradket-tige oder verzweigte Alkyl- oder Alkoxygruppen mit 1—4 C-Atomen substituierten Phenylrest oder einen tertiär- Butylrest,

$R_4$ ein Wasserstoffatom oder einen geradkettigen oder verzweigten Alkylrest mit 1—4 C-Atomen,

$R_5$ ein Wasserstoffatom, einen geradkettigen oder verzweigten, gesättigten oder ungesättigten, gegebenenfalls durch Halogen-, Nitril-, Hydroxy-, Alkoxyreste mit 1—4 C-Atomen in der Alkylkette, Phenyl-, Halogenphenyl oder gegebenenfalls halogenierte Phenoxyreste substituierten Alkyl- oder Oxoalkylrest mit 1—20 C-Atomen, oder einen Cyclohexylrest und

$A^-$ ein pflanzenphysiologisch verträgliches Anion bedeutet, mit Ausnahme der Methyliodide von 6-Methylamino-3-phenylpyridazin und 6-Dimethylamino-3-phenylpyridazin und 6-Amino-3-phenylpyridazin.

2. Pyridaziniumderivate der allgemeinen Formel I nach Anspruch 1, in der

$R_1$ einen gesättigten Alkylrest mit 1 bis 4 C-Atomen oder den Allylrest

$R_2$ einen geradkettigen oder verzweigten Alkylrest mit 1—4 C-Atomen, einen Alkoxy- oder Hydroxyalkylrest mit 1—4 C-Atomen in den Alkylketten oder den Cyclohexylrest oder

$R_1$ und $R_2$ gemeinsam mit dem Stickstoffatom einen 6 oder 7 gliedrigen Ring, der gegebenenfalls ein- oder mehrfach durch Alkylreste mit 1—4 C-Atomen, oder durch einen Benzylrest substituiert ist.

$R_3$ einen gegebenenfalls in ein- oder mehrfach durch Chlor in 3-, 4- oder 5-Stellung oder einen ein- oder mehrfach durch geradkettige oder verzweigte Alkylrest mit 1—4 C-Atomen substituierten Phenylrest,

$R_4$ ein Wasserstoffatom oder einen geradkettigen oder verzweigten Alkylrest mit 1 bis 4 C-Atomen.

$R_5$ einen gesättigten oder ungesättigten Alkylrest mit 1 bis 8 C-Atomen und

$A^-$ ein pflanzenphysiologisch verträgliches Anion bedeutet mit Ausnahme der Methyliodide von 6 Methylamino-3-phenylpyridazin und 6-Dimethylamino-3-phenylpyridazin

3. Pyridaziniumderivate der allgemeinen Formel I nach einem der Ansprüche 1 oder 2 in der

$R_1$ und $R_2$ gemeinsam mit dem Stickstoffatom einen 6 gliedrigen heterocyclischen Ring bilden, der gegebenenfalls ein- oder mehrfach durch Alkylreste mit 1 bis 4 C-Atomen oder durch den Benzylrest substituiert ist,

$R_3$ einen Phenyl, einen p-Chlor- oder p-Methylphenylrest

$R_4$ ein Wasserstoffatom oder einen geradkettigen oder verzweigten Alkylrest mit 1 bis 4 C-Atomen

$R_5$ einen gesättigten oder ungesättigten Alkylrest mit 1—5 C-Atomen und $A^-$ pflanzenphysiologisch verträgliches Anion bedeutet.

4. Verfahren zur Herstellung von Pyridaziniumverbindungen der allgemeinen Formel (I) nach Anspruch (1), dadurch gekennzeichnet, daß man

a) ein Pyridazinverbindung der allgemeinen Formel II

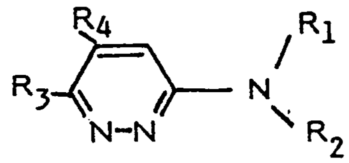

(II)

in der $R_1$, $R_2$, $R_3$, $R_4$ die in Anspruch 1 angegebene Bedeutung haben mit einer Verbindung der allgemeinen Formel

$R_5Y$

in der $R_5$ die in Anspruch 1 angegebene Bedeutung hat und Y ein Halogenatom oder die Reste Methosulfat oder Tosylat bedeutet, oder

b) ein Verbindung der allgemeinen Formel III

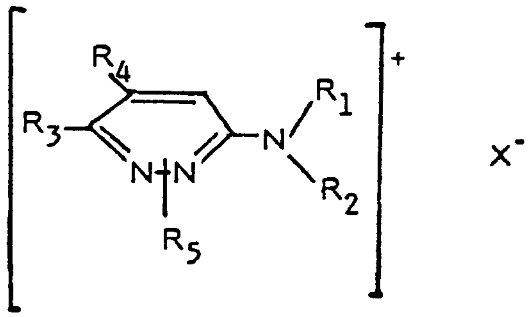

in der $R_1$, $R_2$, $R_3$, $R_4$ und $R_5$ die in Anspruch 1 angegebene Bedeutung haben und X ein Halogenatom bedeutet, mit einem Schwermetallsalz einer anorganischen oder organischen Säure der allgemeinen Formel

Me Z

in der Me ein Schwermetallkation und Z das Anion einer anorganischen oder organischen Säure bedeutet, umsetzt.

5. Fungizide und algizide Mittel gekennzeichnet durch einen Gehalt an mindestens einem Pyridaziniumderivat der allgemeinen Formel

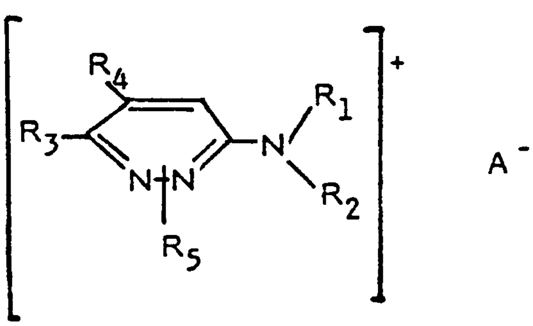

(I)

in der

$R_1$ ein Wasserstoffatom, einen gesättigten oder ungesättigten Alkylrest mit 1—4 C-Atomen,

$R_2$ ein Wasserstoffatom, einen geradkettigen oder verzweigten, gesättigten oder ungesättigten Alkylrest mit 1—10 C-Atomen, einen Cyclohexylrest, einen Alkoxyalkylrest mit 1—4 C-Atomen in der Alkylkette, einen Hydroxyalkylrest mit 1—4 C-Atomen in der Alkylkette, einen gegebenenfalls ein- oder mehrfach durch Halogenatome substituierten Phenylrest oder

$R_1$ und $R_2$ gemeinsam mit dem N-Atom einen 5—7 gliedrigen Ring bilden, der als weiteres Heteroatom Stickstoff oder Sauerstoff enthalten kann und gegebenenfalls durch Alkyl-, Hydroxyalkyl- oder Phenylalkylreste mit 1—4 C-Atomen in der Alkylkette, einen Hydroxyrest oder einen Pyridylrest substituiert ist,

$R_3$ einen gegebenenfalls ein- oder mehrfach durch Halogenatome in 3-, 4-oder 5-Stellung, oder gegebenenfalls ein- oder mehrfach durch geradket-tige oder verzweigte Alkyl- oder Alkoxygruppen mit 1—4 C-Atomen substituierten Phenylrest oder einen tertiär- Butylrest,

$R_4$ ein Wasserstoffatom oder einen geradkettigen oder verzweigten Alkylrest mit 1—4 C-Atomen,

$R_5$ ein Wasserstoffatom, einen geradkettigen oder verzweigten, gesättigten oder ungesättigten, gegebenenfalls durch Halogen-, Nitril-, Hydroxy-, Alkoxyreste mit 1—4 C-Atomen in der Alkylkette, Phenyl-, Halogenphenyl oder gegebenenfalls halogenierte Phenoxyrest substituierten Alkyl- oder Oxoalkylrest mit 1—20 C-Atomen, oder einen Cyclohexylrest und

$A^-$ ein pflanzenphysiologisch verträgliches Anion bedeutet.

6. Fungizide und algizide Mittel nach Anspruch 5 gekennzeichnet durch einen Gehalt an mindestens einem Pyridaziniumderivat der allgemeinen Formel I, in der

$R_1$ einen gesättigten Alkylrest mit 1 bis 4 C-Atomen oder den Allylrest

$R_2$ einen geradkettigen oder verzweigten Alkylrest mit 1—4 C-Atomen, einen Alkoxy- oder Hydroxyalkylrest mit 1—4 C-Atomen in den Alkylketten oder den Cyclohexylrest oder

$R_1$ und $R_2$ gemeinsamt mit dem Stickstoffatom einen 6 oder 7 gliedrigen Ring, der gegebenenfalls ein- oder mehrfach durch Alkylreste mit 1—4 C-Atomen, oder durch einen Benzylrest substituiert ist

$R_3$ einen gegebenenfalls ein- oder mehrfach durch Chlor in 3-, 4- oder 5-Stellung substituierten oder einen ein- oder mehrfach durch geradkettige oder verzweigte Alkylreste mit 1—4 C-Atomen substituierten Phenylrest,

$R_4$ ein Wasserstoffatom oder einen geradkettigen oder verzweigten Alkylrest mit 1—4 C-Atomen

$R_5$ einen gesättigten oder ungesättigten Alkylrest mit 1—8 C-Atomen und

$A^-$ ein pflanzenphysiologisch verträgliches Anion bedeutet.

7. Fungizide und algizide Mittel nach einem der Ansprüche 5 oder 6 gekennzeichnet durch einen Gehalt an midestens einem Pyridaziniumderivat der allgemeinen Formel I, in der

$R_1$ und $R_2$ gemeinsam mit dem Stickstoffatom einen 6 gliedrigen heterocyclischen Ring bilden, der gegebenenfalls ein- oder mehrfach durch Alkylreste mit 1 bis 4 C-Atome oder durch den Benzylrest substituiert ist.

$R_3$ einen Phenyl-, einen p-Chlor- oder p-Methylphenylrest

$R_4$ ein Wasserstoffatom oder einen geradkettigen oder verzweigten Alkylrest mit 1 bis 4 C-Atomen

$R_5$ einen gesättigten oder ungesättigten Alkylrest mit 1—5 C-Atomen und

$A^-$ ein pflanzenphysiologisch verträgliches Anion bedeutet.

8. Verfahren zur Bekämpfung von Schadorganismen, dadurch gekennzeichnet, daß man Pyridaziniumderivate der allgemeinen Formel I, in der die Reste $R_1$, $R_2$, $R_3$, $R_4$, $R_5$ und A die in Anspruch 5 angegebene Bedeutung haben, auf Schadorganismen oder deren Lebensraum einwirken läßt.

9. Verwendung von Pyridaziniumderivaten der allgemeinen Formel I, in welcher die Reste $R_1$, $R_2$, $R_3$, $R_4$, $R_5$ und A die in Anspruch 5 angegebene Bedeutung haben, zur Bekämpfung von Schadorganismen.

10. Verfahren zur Herstellung von algiziden und fungiziden Mitteln, dadurch gekennzeichnet, daß man Pyridaziniumderivate der allgemeinen Formel I, in der $R_1$, $R_2$, $R_3$, $R_4$, $R_5$ und A die in Anspruch 5 angegebene Bedeutung haben, mit Streckmitteln und/oder oberflächenaktiven Mitteln vermischt.

**Revendications**

1. Dérivés pyridazinium de formule générale I

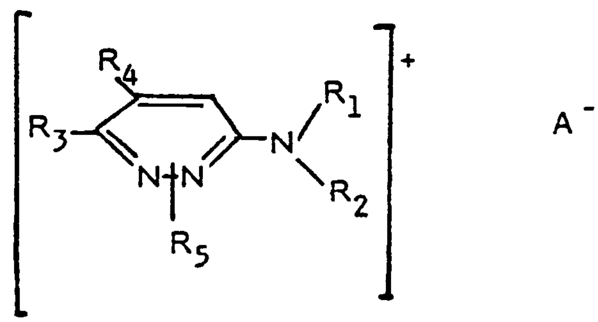

(I)

dans laquelle:

$R_1$ est un atome d'hydrogène, un reste alkyle saturé ou non comprenant 1 à 4 atomes de carbone,

$R_2$ est un atome d'hydrogène, un reste alkyle de 1 à 10 atomes de carbone, ramifié ou non, et saturé ou non, un reste cyclohexyle, un reste alcoxyalkyle de 1 à 4 atomes de carbone dans la chaîne alkyle, un reste hydroxyalkyle de 1 à 4 atomes de carbone dans la chaîne alkyle un reste phényle substitué, éventuellement par un ou plusieurs atomes d'halogène, ou bien,

$R_1$ et $R_2$ constituent avec l'azote un cycle de 5 à 7 chaînons renfermant un autre hétéroatome constitué par l'azote ou l'oxygène, substitué le cas échéant par restes alkyles, hydroxyalkyles, phénylalkyles comprenant 1 à 4 atomes de carbone dans la chaîne alkyle, un reste hydroxy, ou bien un groupe pyridyle, sous réserve que les substituants, ne sont pas représentés par un groupe hydroxy si $R_1$ et $R_2$ constituent avec l'atome d'azote un cycle à 6 chaînons,

$R_3$ est un groupe phényle substitué le cas échéant par un ou plusieurs atomes d'halogène dans les

positions 3, 4 et 5, ou bien par un ou plusieurs groupes alkyle ou alcoxy de 1 à 4 atomes de carbone à chaîne droite ou ramifiée, ou bien un groupe tertiobutyle,

$R_4$ est un atome d'hydrogène ou bien un groupe alkyle de 1 à 4 atomes de carbone à chaîne droite ou ramifiée,

$R_5$ est un atome d'hydrogène, un groupe alkyle ou oxoalkyle de 1 à 20 atomes de carbone à chaîne droite ou ramifiée, saturé ou non, substitué le cas échéant par des halogènes, des nitriles, des hydroxy, des restes alcoxy de 1 à 4 atomes de carbone dans la chaîne alkyle, des phenylies, des phényles halogénes ou des restes phenoxy halogenés, ou bien un reste cyclohexyle et.

A- signifie un anion admissible physiologiquement par les plantes.

à l'exception du methyliodure de 6-méthylamino-3-phenyl pyridazine, du 6-diméthylamino-3-phényl-pyridazine et du 6-amino-3-phénylpyridazine.

2. Dérivés pyridazinium de formule générale I selon la revendication 1, dans laquelle:

$R_1$ est un reste alkyle saturé ou non comprenant 1 à 4 atomes de carbone, ou un reste allyle,

$R_2$ est un reste alkyle de 1 à 4 atomes de carbone, ramifié ou non, un reste alcoxyalkyle ou hydroxyalkyle de 1 à 4 atomes de carbone dans la chaîne alkyle, ou un reste cyclohexyle, ou bien,

$R_1$ et $R_2$ constituent avec l'azote un cycle de 6 ou 7 chaînons substitué le cas échéant une ou plusieurs fois par un reste alkyle de 1 à 4 atomes de carbone, ou un reste benzyle,

$R_3$ est un groupe phényle substitué le cas échéant par un ou plusieurs atomes d'halogène dans les positions 3, 4 et 5, ou bien par un ou plusieurs groupes alkyle de 1 à 4 atomes de carbone à chaîne droite ou ramifiée.

$R_4$ est un atome d'hydrogène ou bien un groupe alkyle de 1 à 4 atomes de carbone à chaîne droite ou ramifiée.

$R_5$ est un groupe alkyle saturé ou non, de 1 à 8 atomes de carbone,

A- signifie un anion admissible physiologiquement par les plantes, à l'exception du iodure de méthyle de 6-méthylamino-3-phénylpyridazine et du 6-diméthylamino-3-phénylpyridazine.

3. Dérivés pyridazinium de formule générale I selon la revendication 1 ou 2, dans laquelle,

$R_1$ et $R_2$ constituent avec l'atome d'azote, un hétérocycle à 6 chaînons substitué le cas échéant une ou plusieurs fois par un groupe alkyle de 1 à 4 atomes de carbone ou un reste benzyle,

$R_3$ est un reste phényle, p-chloro ou p-méthylphényle.

$R_4$ signifie un atome d'hydrogène, ou un reste alkyle de 1 à 4 atomes de carbone, à chaîne droite ou ramifiée,

$R_5$ signifie un reste alkyle de 1 à 5 atomes de carbone, saturé ou non saturé,

A- signifie un anion admissible physiologiquement par les plantes.

4. Procédé de préparation des composés pyridazinium de formule générale I selon la revendication 1, caractérisé en ce qu'on fait réagir:

a) un composé pyridazine de formule générale II

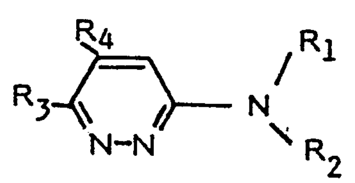

(II)

dans laquelle:

$R_1$, $R_2$, $R_3$ $R_4$ ont les significations indiquées ci-dessus dans la revendication 1 avec un composé de formule générale:

$$R_5Y$$

dans laquelle

$R_5$ a la signification susindiquée et Y représente un atome d'halogène, ou un reste méthosulfate ou tosylate, ou bien,

b) un composé de formule générale III

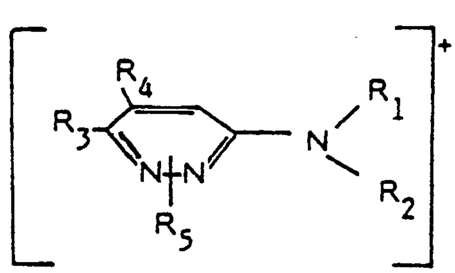

$X^-$ (III)

dans laquelle:

$R_1$, $R_2$, $R_3$, $R_4$ et $R_5$ ont la signification indiquée dans la revendication 1, X signifie un atome d'halogène, avec un sel de métal lourd d'un acide organique ou non organique de formule:

Me Z

dans laquelle Me est un cation métallique lourd et Z l'anion d'un acide non organique ou organique.

5. Composition fongicide et algicide, caractérisée en ce qu'elle renferme au moins un dérivé pyridazinium de formule générale:

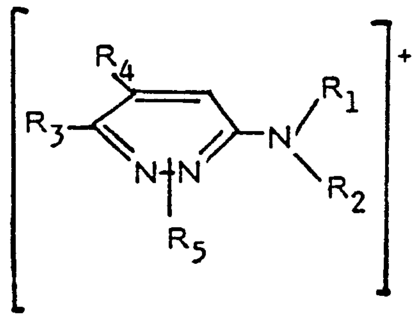

$A^-$ (I)

dans laquelle:

$R_1$ est un atome d'hydrogène, un reste alkyle saturé ou non comprenant 1 à 4 atomes de carbone,

$R_2$ est un atome d'hydrogène, un reste alkyle de 1 à 10 atomes de carbone, ramifié ou non, et saturé ou non, un reste cyclohexyle, un reste alcoxyalkyle de 1 à 4 atomes de carbone dans la chaîne alkyle, un reste hydroxyalkyle de 1 à 4 atomes de carbone dans la chaîne alkyle un reste phényle substitué, éventuellement par un ou plusieurs atomes d'halogène, ou bien,

$R_1$ et $R_2$ constituent avec l'azote un cycle de 5 à 7 chaînons renfermant un autre hétéroatome constitué par l'azote ou l'oxygène, substitué le cas échéant par restes alkyles, hydroxyalkyles, phénylalkyles comprenant 1 à 4 atomes de carbone dans la chaîne alkyle, un reste hydroxy, ou bien un groupe pyridyle, sous réseve que les substituants ne sont pas représentés par un groupe hydroxy si $R_1$ et $R_2$ constituent avec l'atome d'azote un cycle à 6 chaînons,

$R_3$ est un groupe phényle substitué le cas échéant par un ou plusieurs atomes d'halogène dans les positions 3, 4 et 5, ou bien par un ou plusieurs groupes alkyle ou alcoxy de 1 à 4 atomes de carbone à chaîne droite ou ramifiée, ou bien un groupe tertiobutyle,

$R_4$ est un atome d'hydrogène ou bien un groupe alkyle de 1 à 4 atomes de carbone à chaîne droite ou ramifiée,

$R_5$ est un atome d'hydrogène, un groupe alkyle ou oxoalkyle de 1 à 20 atomes de carbone à chaîne droite ou ramifiée, saturé ou non, substitué le cas échéant par des halogènes, des nitriles, des hydroxy, des restes alcoxy de 1 à 4 atomes de carbone dans la chaîne alkyle, des phenyles, des phényles halogènes ou des restes phénoxy halogénés, ou bien un rest cyclohexyle et.

A- signifie un anion admissible physiologiquement par les plantes.

6. Composition fongicide et algicide selon la revendication 5, caractérisé en ce qu'elle renferme au moins un dérivé pyridazinium de formule générale I dans laquelle:

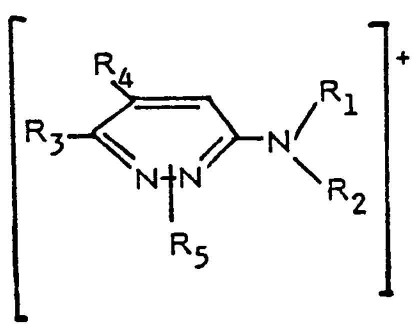

$A^-$ (I)

$R_1$ est un reste alkyle saturé ou non comprenant 1 à 4 atomes de carbone, ou un reste allyle,

$R_2$ est un reste alkyle de 1 à 4 atomes de carbone, ramifié ou non, un reste alcoxyalkyle ou hydroxyalkyle de 1 à 4 atomes de carbone dans la chaîne alkyle, ou un reste cyclohexyle, ou bien,

$R_1$ et $R_2$ constituent avec l'azote un cycle de 6 ou 7 chaînons substitué le cas échéant une ou plusieurs fois par un reste alkyle de 1 à 4 atomes de carbone, ou un reste benzyle,

$R_3$ est un groupe phényle substitué le cas échéant par un ou plusieurs atomes d'halogène dans les

positions 3, 4 et 5, ou bien par un ou plusieurs groupes alkyle de 1 à 4 atomes de carbone à chaîne droite ou ramifiée,

$R_4$ est un atome d'hydrogène ou bien un groupe alkyle de 1 à 4 atomes de carbone à chaîne droite ou ramifiée,

$R_5$ est un groupe alkyle saturé ou non, de 1 à 8 atomes de carbone,

A- signifie un anion admissible physiologiquement par les plantes.

7. Composition fongicide et algicide selon la revendication 5 ou 6, caractérisée en ce qu'elle renferme au moins un compose pyridazinium de formule générale 1, dans laquelle:

$R_1$ et $R_2$ constituent avec l'atome d'azote, un hétérocycle à 6 chaïnons substitué, le cas écheant une ou plusieurs fois par un groupe alkyle de 1 à 4 atomes de carbone ou un reste benzyle.

$R_3$ est un reste phényle, p-chloro ou p-methylphényl.

$R_4$ signifie un atome d'hydrogene, ou un reste alkyle de 1 à 4 atomes de carbone, a chaîne droite ou ramifiée,

$R_5$ signifie un reste alkyle de 1 à 5 atomes de carbone, saturé ou non sature.

A- signifie un anion admissible physiologiquement par les plantes.

8. Procedé de lutte contre les organismes destructeurs, caractérisé en ce qu'on fait réagir les dérivés pyridazinium de formule générale I dans laquelle les restes $R_1$ à $R_5$ et A ont la signification indiquée dans la revendication 5 sur les organismes destructeurs ou bien dans leur espace vital.

9. Utilisation des dérivés pyridazinium de formule générale I dans laquelle les restes $R_1$ à $R_5$ et A ont la signification indiquée dans la revendication 5 pour combattre les organismes destructeurs.

10. Procédé d'obtention de compositon algicide et fongicide, caractérisé en ce qu'on mélange les dérivés pyridazinium selon la formule générale I dans laquelle $R_1$ à $R_5$ et A ont la signification indiquée dans la revendication 5 avec des diluants et/ou des substances à surface externe active.

## Claims

1. A pyridazinium derivative of the formula I

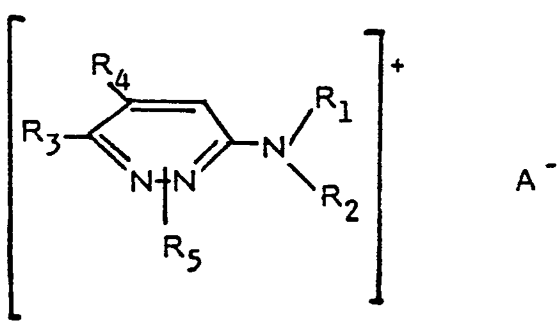

(I)

in which $R_1$ denotes a hydrogen atom or a saturated or unsaturated alkyl radical with 1—4 C atoms, $R_2$ denotes a hydrogen atom, a straight-chain or branched, saturated or unsaturated alkyl radical with 1—10 C atoms, a cycloalkyl radical, an alkoxyalkyl radical with 1—4 C atoms in the alkyl chain, a hyroxyalkyl radical with 1—4 C atoms in the alkyl chain or a phenyl radical which is optionally monosubstituted or polysubstituted by halogen atoms, or $R_1$ and $R_2$, together with the N atom, form a 5- to 7-membered ring, which can contain nitrogen or oxygen as a further hetero atom and is optionally substituted by alkyl, hydroxyalkyl or phenylalkyl radicals with 1—4 C atoms in the alkyl chain, a hydroxyl radical or a pyridyl radical, with the proviso that the substituent is not hydroxyl when $R_1$ and $R_2$, together with the N-atom, form a 6-membered ring, $R_3$ denotes a phenyl radical which is optionally mono- or polysubstituted by halogen atoms in the 3-, 4- or 5-position or optionally mono- or polysubstituted by straight-chain or branched alkyl or alkoxy groups with 1—4 C atoms, or denotes a tert.-butyl radical, $R_4$ denotes a hydrogen atom or a straight-chain or branched alkyl radical with 1—4 C atoms, $R_5$ denotes a hydrogen atom, a straight-chain or branched, saturated or unsaturated alkyl or oxoalkyl radical which has 1—20 C atoms and is optionally substituted by halogen, nitrile, hydroxyl, alkoxy radicals with 1—4 C atoms in the alkyl chain, phenyl, halogenophenyl or optionally halogenated phenoxy radicals, or denotes a cycloalkyl radical and A denotes an anion which is physiologically tolerated by plants, with the exception of the methyliodides of 6-methylamino-3-phenylpyridazine, 6-dimethylamino-3-phenylpyridazine and 6-amino-3-phenylpyridazine.

2. A pyridazinium derivative of the formula I according to Claim 1, in which $R_1$ denotes a saturated alkyl radical with 1 to 4 C atoms or the allyl radical and $R_2$ denotes a straight-chain or branched alkyl radical with 1—4 C atoms, an alkoxy or hydroxyalkyl radical with 1—4 C atoms in the alkyl chains or the cyclohexyl radical, or $R_1$ or $R_2$, together with the nitrogen atom, denote a 6-membered or 7-membered ring, which is optionally mono- or polysubstituted by alkyl radicals with 1—4 C atoms or substituted by a benzyl radical, $R_3$ denotes a phenyl radical which is optionally mono- or polysubstituted by chlorine in the 3-, 4- or 5-position or a phenyl radical which is mono- or polysubstituted by straight-chain or branched alkyl radicals

with 1—4 C atoms, $R_4$ denotes a hydrogen atom or a straight-chain or branched alkyl radical with 1 to 4 C atoms, $R_5$ denotes a saturated or unsaturated alkyl radical with 1 to 8 C atoms and A denotes an anion which is physiologically tolerated by plants, with the exception of the methyl iodides of 6-methylamino-3-phenylpyridazine and 6-dimethylamino-3-phenylpyridazine.

3. A pyridazinium derivative of the formula I according to any one of Claims 1 and 2, in which $R_1$ and $R_2$, together with the nitrogen atom, for a 6-membered heterocyclic ring, which is optionally mono- or polysubstituted by alkyl radicals with 1 to 4 C atoms or substituted by the benzyl radical, $R_3$ denotes a phenyl radical or a p-chloro- or p-methylphenyl radical, $R_4$ denotes a hydrogen atom or a straight-chain or branched alkyl radical with 1 to 4 C atoms, $R_5$ denotes a saturated or unsaturated alkyl radical with 1—5 C atoms and A denotes an anion which is physiologically tolerated by plants.

4. A process for the preparation of a pyridazinium compound of the formula I according to Claim 1, characterized in that

a) a pyridazine compound of the formula II

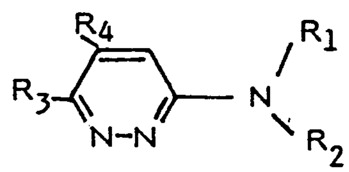

(II)

in which $R_1$, $R_2$, $R_3$ and $R_4$ have the meaning given in Claim 1, is reacted with a compound of the formula $R_5Y$ in which $R_5$ has the meaning given in Claim 1 and Y denotes an halogen atom or the methosulphate or tosylate radicals, or

b) a compound of the formula III

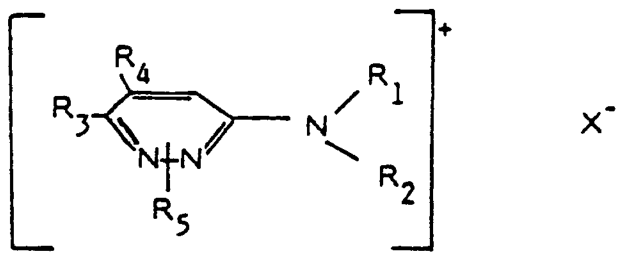

(III)

in which $R_1$, $R_2$, $R_3$, $R_4$ and $R_5$ have the meaning given in Claim 1 and X denotes a halogen atom, is reacted with a heavy metal salt of an inorganic or organic acid of the formula MeZ in which Me denotes a heavy metal cation and Z denotes the anion of an inorganic or organic acid.

5. A fungicidal and algicidal agent, characterized in that is contains at least one pyridazinium derivative of the formula

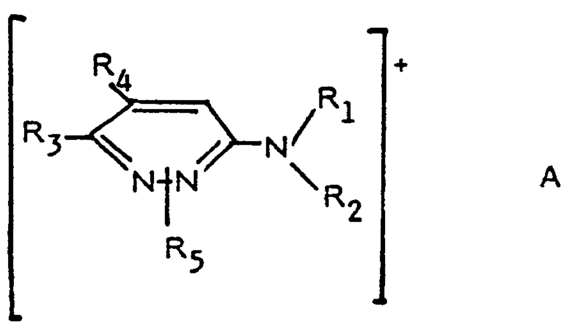

(I)

in which $R_1$ denotes a hydrogen atom or a saturated or unsaturated alkyl radical with 1—4 C atoms, $R_2$ denotes a hydrogen atom, a straight-chain or branched, saturated or unsaturated alkyl radical with 1—10 C atoms, a cycloalkyl radical, an alkoxyalkyl radical with 1—4 C atoms in the alkyl chain, a hydroxyalkyl radical with 1—4 C atoms in the alkyl chain or a phenyl radical which is optionally monosubstituted or polysubstituted by halogen atoms, or $R_1$ and $R_2$, together with the N atom, form a 5- to 7-membered ring, which can contain nitrogen or oxygen as a further hetero atom and is optionally substituted by alkyl, hydroxyalkyl or phenylalkyl radicals with 1—4 C atoms in the alkyl chain, a hydroxyl radical or a pyridyl radical, $R_3$ denotes a phenyl radical which is optionally mono- or polysubstituted by halogen atoms in the 3-, 4- or 5-position or optionally mono- or polysubstituted by straight-chain or branched alkyl or alkoxy groups with 1—4 C atoms, or denotes a tert.-butyl radical, $R_4$ denotes a hydrogen atom or a straight-chain or branched alkyl radical with 1—4 C atoms, $R_5$ denotes a hydrogen atom, a straight-chain or branched, saturated or unsaturated alkyl or oxoalkyl radical which has 1—20 C atoms and is optionally substituted by halogen,

nitrile, hydroxyl, alkoxy radicals with 1—4 C atoms in the alkyl chain, phenyl, halogenophenyl or optionally halogenated phenoxy radicals, or denotes a cyclohexyl radical and A denotes an anion which is physiologically tolerated by plants.

6. A fungicidal and algicidal agent according to Claim 5, characterized in that it contains at least one pyridazinium derivative of the formula I in which $R_1$ denotes a saturated alkyl radical with 1 to 4 C atoms or the allyl radical and $R_2$ denotes a straight-chain or branched alkyl radical with 1—4 C atoms, an alkoxy or hydroxyalkyl radical with 1—4 C atoms in the alkyl chains or the cyclohexyl radical, or $R_1$ and $R_2$, together with the nitrogen atom, form a 6-membered or 7-membered ring, which is optionally mono- or polysubstituted by alkyl radicals with 1—4 C atoms or substituted by a benzyl radical, $R_3$ denotes a phenyl radical which is optionally mono- or polysubstituted by chlorine in the 3-, 4- or 5-position or a phenyl radical which is mono- or polysubstituted by straight-chain or branched alkyl radicals with 1—4 C atoms, $R_4$ denotes a hydrogen atom or a straight-chain or branched alkyl radical with 1 to 4 C atoms, $R_5$ denotes a saturated or unsaturated alkyl radical with 1—8 C atoms and A denotes an anion which is physiologically tolerated by plants.

7. A fungicidal and algicidal agent according to any one of Claims 5 and 6, characterized in that it contains pyridazinium derivative of the formula I in which $R_1$ and $R_2$, together with the nitrogen atom, form a 6-membered heterocyclic ring, which is optionally mono- or polysubstituted by alkyl radicals with 1 to 4 C atoms or substituted by the benzyl radical, $R_3$ denotes a phenyl radical or a p-chloro- or p-methylphenyl radical, $R_4$ denotes a hydrogen atom or a straight-chain or branched alkyl radical with 1 to 4 C atoms, $R_5$ denotes a saturated or unsaturated alkyl radical with 1—5 C atoms and $A^-$ denotes an anion which is physiologically tolerated by plants.

8. A method of combating harmful organisms, characterized in that a pyridazinium derivative of the formula I in which the radicals $R_1$, $R_2$, $R_3$, $R_4$, $R_5$ and A have the meaning given in Claim 5 is allowed to act on harmful organisms or their environment.

9. The use of a pyridazinium derivative of the formula I in which the radicals $R_1$, $R_2$, $R_3$, $R_4$, $R_5$ and A have the meaning given in Claim 5 for combating harmful organisms.

10. A process for the preparation of an algicidal and fungicidal agent, characterized in that a pyridazinium derivative of the formula I in which $R_1$, $R_2$, $R_3$, $R_4$, $R_5$ and A have the meaning given in Claim 5 is mixed with extenders and/or surface-active agents.